(19) Europäisches Patentamt
European Patent Office
Office européen des brevets

(11) **EP 3 253 279 B1**

(12) **FASCICULE DE BREVET EUROPEEN**

(45) Date de publication et mention
de la délivrance du brevet:
**12.04.2023 Bulletin 2023/15**

(21) Numéro de dépôt: **16707857.5**

(22) Date de dépôt: **01.02.2016**

(51) Classification Internationale des Brevets (IPC):
**A61B 5/00** *(2006.01)* **A61B 6/14** *(2006.01)*
**A61B 8/00** *(2006.01)*

(52) Classification Coopérative des Brevets (CPC):
**A61B 5/0088; A61B 5/6803; A61B 5/7425;**
**A61B 6/022; A61B 6/14; A61B 6/462; A61B 6/463;**
**A61B 6/466; A61B 6/5229; A61B 8/12;**
**A61B 8/462; A61B 34/20; A61B 90/361;**
**A61B 90/37; A61C 1/082;** (Cont.)

(86) Numéro de dépôt international:
**PCT/FR2016/050206**

(87) Numéro de publication internationale:
**WO 2016/124847 (11.08.2016 Gazette 2016/32)**

(54) **DISPOSITIF DE VISUALISATION DE L'INTÉRIEUR D'UNE BOUCHE**

VORRICHTUNG ZUM ANZEIGEN DES INNERN EINES MUNDES

DEVICE FOR VIEWING THE INSIDE OF A MOUTH

(84) Etats contractants désignés:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priorité: **03.02.2015 FR 1550817**

(43) Date de publication de la demande:
**13.12.2017 Bulletin 2017/50**

(73) Titulaire: **Duret, François**
**11560 Fleury d'Aude (FR)**

(72) Inventeur: **Duret, François**
**11560 Fleury d'Aude (FR)**

(74) Mandataire: **Rhein, Alain**
**Cabinet BREV & SUD**
**55 Avenue Clément Ader**
**34170 Castelnau-le-Lez (FR)**

(56) Documents cités:
**US-A- 6 006 126 US-A1- 2004 119 662**
**US-A1- 2009 124 890 US-A1- 2012 056 993**

(52) Classification Coopérative des Brevets (CPC):
(Cont.)
**A61C 1/084; G06T 7/32;** A61B 6/145;
A61B 2090/365; A61B 2562/17; Y02A 90/10

**Description**

La présente invention concerne un dispositif de visualisation de l'intérieur d'une bouche, notamment un dispositif permettant de voir une partie interne située sous une surface externe d'un organe disposé dans la bouche.

Les dispositifs connus ayant pour objet une visualisation dans le domaine des traitements dentaires des parties invisibles sous gingivales, coronaires, radiculaires ou osseuses comprennent les appareils suivants :

- Un émetteur de rayons pénétrants tel qu'un appareil de radiologie travaillant en 2D (radiographie classique ou radiovisiographie), en 2D 1/2 ou en 3D (scanner, technique de radiographie volumique par faisceau conique (usuellement appelée cone beam), panoramique ou ortho-pantomographe) ou un appareil IRM, un appareil ultrasonique, un appareil travaillant avec des rayonnements térahertz ou un appareil travaillant avec les techniques dérivées de l'interférométrie holographique (OCT) ;
- Eventuellement une caméra endobuccale de prise d'empreinte par des moyens optiques (rayonnement allant du bleu profond au rayon X, voire ultrasonique) utilisant ou non une lumière projetée structurée ; et
- Un écran de visualisation déportée permettant de voir, d'une part la vue de la modélisation issue de l'appareil émetteur de rayons pénétrants et d'autre part la modélisation obtenue à la suite du balayage effectué à l'aide de la caméra endobuccale s'affichant après que le clinicien ait procédé à l'empreinte optique.

Les appareils de radiologie utilisés dans les traitements dentaires peuvent se diviser en deux grandes catégories, ceux se trouvant à proximité de l'unit dentaire et ceux qui sont déportés.

Dans la première catégorie, nous trouvons des appareils utilisant des supports argentiques, phosphoriques ou numériques (radiovisiographie ou RVG).

Si les appareils argentiques sont de moins en moins utilisés, il n'en n'est pas de même des deux autres car ils permettent de numériser indirectement (supports phosphoriques) ou directement (RVG) l'image radiologique pixélisée obtenue des tissus osseux par transparence aux rayons X. Dans les deux cas, l'image obtenue est numérisée en niveau de gris et affichée en 2D sur un écran à proximité du praticien en noir et blanc ou en couleurs virtuelles reconstituées. Cette image lui permet de connaître l'état sous gingival des tissus osseux mais aussi des couronnes et des racines des dents.

Le clinicien report et fait correspondre intuitivement les formes visualisées vues à l'écran 2D sur les parties visibles dans la bouche de son patient. Cela lui permet d'avoir une idée très approximative de savoir la forme et la longueur d'une racine, de savoir s'il existe des images pathologiques et d'imaginer la position des nerfs et des gros vaisseaux sanguins.

S'il veut suivre aussi dans le temps si son traitement est efficace ou non il devra effectuer plusieurs clichés successifs.

Avec l'apparition d'une dentisterie plus exigeante, en particulier s'adressant aux traitements en parodontologie et en implantologie, a été utilisé des appareils plus complexes représentant une deuxième catégorie. Ces appareils sont rarement dans le cabinet dentaire mais ils permettent au dentiste d'avoir une vue générale de toute la bouche en 2D, 2D 1/2 voire même 3D s'il utilise des rayonnements par résonnance (IRM).

Dans cette catégorie nous trouvons depuis une trentaine d'année les scanners buccaux (pantographes, panoramiques) donnant des images en 2D de toute l'arcade sur un seul cliché, les tomodensitomètres donnant des images 2D 1/2 permettant grâce aux différents plans de voxels, de reconstituer une fausse image 3D (scanner) et plus récemment les cone beam associant les avantages du scanner traditionnel et des tomodensitomètre donnant une image 2D 1/2 très rapide et beaucoup plus précise des tissus osseux.

Ces dernières images sont très utilisées en implantologie où le praticien doit connaître exactement la position des organes sous-jacents comme les sinus et les différentes structures osseuses lorsqu'il prépare le site de réception de son futur implant.

Dans tous les cas, ces images spatiales 2D 1/2 (ou fausse 3D) sont représentées sur un écran déporté 2D lui permettant de les faire bouger dans les trois plans de l'espace et de savoir où se situes les zones intéressantes ou les zones à risques.

Certain praticiens, enfin, utilisent de vraies images 3D sous IRM, mais cela est encore rare et très coûteux. Dans ce cas, encore, la visualisation se fera sur un moniteur déporté.

Récemment, et devant l'imprécision de l'image radiologique, certains cliniciens ont décidé d'associer à l'image radiologique imprécise (100 à 200 microns) une image de la partie externe beaucoup plus précise (10 à 30 microns) obtenue à l'aide de caméra endobuccale d'empreinte optique. Par fusion entre la première image et la seconde, ils obtiennent sur l'écran 2D déporté une vue confondue des tissus et organes sous-jacents et de l'empreinte optique des dents et de la gencive.

Malheureusement si la connaissance de la proximité d'un organe sous-jacent est acceptable à une centaine de micron près, il n'en n'est pas de même de la précision d'une couronne ou du fût d'implant qui doit être connu à une dizaine de micron près.

S'ils utilisent les systèmes décrit précédemment pour la vue sous gingivale, ils leurs faut en plus une caméra d'empreinte optique pour avoir une vue externe suffisamment précise.

Aujourd'hui découlant directement des travaux de l'inventeur, existent différents types de méthode de prise d'empreinte optique endobuccale en cabinet dentaire que l'on peut fusionner sur une image radiologique. Nous y trouvons :

- Celles projetant sur la dent une lumière structurée qui peut être un point, une ligne voire une grille complète. Elles sont très largement connues depuis plusieurs décennies et sont très bien décrites dans « light sectioning with large depth and high resolution » in Appl. Opt. 27 (1988). Elles peuvent utiliser, par exemple, des projections de grilles à pas variables (« numérical stéréo caméra » SPIE vol 283 3-D, 1981), le principe de la phase profilométrique (US 5.092.022 et US 4.952.149) dont la plus connue est le CEREC (Sirona GmbH), celui associant la projection de frange et les variations de phases de la société Hint-Els ou le principe confocale parallèle comme l'Itéro (US.0109559) de Cadent.
- Celles n'utilisant pas la projection de lumière active ou structurée mais l'interférométrie stéréoscopique. C'est le cas de la caméra Lava AWS (US 7.372.642) ou de la caméra Condor (US 8.520.925) .

Si nous pouvons dire que tous ces travaux et inventions ont conduit à de nombreuses réalisations et à plus de vingt systèmes commercialement disponibles (le fil dentaire n° 63, mai 2011, « la grande aventure de la CFAO à l'IDS de Cologne » 14-26), aucun n'a proposé une solution originale permettant de visualiser l'empreinte des parties visibles et invisibles directement en bouche pendant et après leurs réalisations.

Toutes ces méthodes décrites, mises en œuvre dans les cabinets dentaires ou dans un autre local pour les gros appareils de radiologie, utilisent le même système de visualisation : un écran déporté proche ou éloigné de l'opérateur. Quelle que soit la complexité de ces appareils, à toutes les caméras ou appareils de radiologie que nous venons de décrire est associé un écran. Il peut surmonter un chariot, être connecté ou dépendant (all in one) d'un ordinateur ou être tout ou partie d'un ordinateur portable ou d'une tablette.

S'il s'agit d'un moniteur informatique (vidéo, plasma, LCD, ou à LED). L'écran, est spécifique de l'application, radiologique ou issue de la prise de l'empreinte optique. Quelque fois il associe les deux méthodes (Planmeca, Carestream) en affichant dans deux fenêtres distinctes la vue vidéo issue de la caméra et la vue modélisée issue du traitement numérique radiologique et/ou endobuccale.

Sur ce même écran peut s'afficher l'interactif du praticien lui permettant de remplir les informations se rapportant au patient : les caractéristiques médicales et les soins à faire ou déjà fait. On appelle cela la fiche patient. Dans ce cas ce n'est pas un problème d'afficher ces informations sur un écran déporté dans la mesure où les éléments que contient cette fiche sont rarement remplis en cours d'actes ou ne nécessites pas d'être visualisés durant ceux-ci. Même si cela a déjà conduit à la réalisation d'application en réalité augmentée, c'est pour nous de peu d'intérêt pour la santé du patient. Il n'en n'est pas de même de l'affichage de ses données physiologiques durant l'intervention, comme nous le verrons dans les applications accessoires de notre invention.

L'unité centrale de traitement numérique réunit et traite les informations venues de la caméra endobuccale et des appareils de radiologie puis les affichent sur les écrans de visualisation.

Nous comprenons immédiatement que le premier problème que rencontre l'opérateur est de devoir regarder sur un ou plusieurs écrans déportés la vue radiologique et celle issue de sa caméra endobuccale. S'il utilise un support argentique il n'a pas d'autre possibilité d'utiliser un négatoscope. Ceci l'oblige à détourner son regard et à ne jamais avoir de correspondance précise entre son espace clinique, qui est ce qu'il voit dans la bouche de son patient, et la zone sous gingivale connue radiologiquement et affichée sur le moniteur.

Nous comprenons pourquoi le clinicien doit sans arrêt déporter son regard de son champ opératoire à l'image déportée.

De plus si des indications en réalité augmentées lui sont données sur l'écran déporté, il doit faire, non seulement l'effort de déplacer son regard de son champ opératoire vers le moniteur, mais aussi celui de transposer par l'esprit et virtuellement ces indications et informations visibles sur l'écran déporté 2D jusqu'au champ opératoire avec le risque d'être imprécis ou de mal le faire.

Ceci est extrêmement aléatoire, d'autant plus que la seule région correspondante à un volume commun entre la partie visible et la partie sous gingivale permettant une corrélation par l'esprit est, dans la vue radiologique affichée en 2D sur l'écran alors qu'en bouche sa vision est tridimensionnelle. L'opération est tellement imprécise en implantologie que les cliniciens doivent utiliser des guides se fixant sur les dents pour que leurs forêts ne lèsent pas les tissus sous-jacents.

Nous comprenons facilement que de voir indirectement le cours et le résultat de son travail est dangereux pour le patient, imprécis, incomplet et extrêmement dommageable dans la pratique quotidienne. Nous pouvons résumer les problèmes qu'entraine ce mode d'affichage sur un écran déporté de la manière suivante :

- Cela oblige ce dernier à déplacer en permanence sont regard entre la partie du corps sur laquelle il travaille et l'écran déporté. En effet si le praticien souhaite suivre l'évolution de son travail endodontique ou en chirurgie, il doit quitter la vue de la zone du corps sur laquelle il travail et regarder sur son écran vidéo (moniteur) ou numérique pour deviner où se situe son travail.
- Ce déplacement peut entraîner des mouvements de ses mains néfastes, imprécis et incontrôlés durant son travail, problème d'autant plus important s'il travaille longtemps (fatigue).
- Ce déplacement est dangereux car son regard quitte régulièrement le champ opératoire au risque de provoquer une blessure dans la bouche ou le corps du patient ou de fracturer ses instruments.
- Cela est aussi très fatigant car l'existence d'un écran déporté oblige à une gymnastique oculaire à un rythme très élevé. Il est ainsi possible d'avoir plus de 20 mouvements aller-et-retour de ses yeux par minutes.
- Cela exclu toute information complémentaire directement corrélée sur le champ visualisé comme le permet aujourd'hui la réalité augmentée. Le fait de n'avoir aucune corrélation entre la vue réelle et l'information issue de la réalité augmentée par exemple sur un écran déporté exclu tout temps réel et toute information précise dans le champ opératoire. Même si cette information apparait sur l'écran déporté, la visualisation ne sera jamais en temps réel et le geste du clinicien précisément positionnée dans le champ de travail.

Cette action est imprécise : nous voyons que s'il est possible de voir les tissus sous-jacents sur un écran déporté, la visualisation directe de son travail n'est jamais sécurisé car le fait de déplacer son regard et de modifier lors de son travail la zone d'action clinique rend difficile la corrélation entre les deux observations. Aucune corrélation vraie entre cette représentation RX et le champ de travail n'existe du fait de l'utilisation de l'écran déporté. Il en est de même pour toute information issue des logiciels de réalité augmentée reportée sur l'écran déporté.

Cette opération est insuffisante : le rayonnement RX produit une visualisation 2D ou 2D 1/2 reportée sur un écran 2D ce qui rend particulièrement difficile voire impossible l'estimation de ce qui a été radiographié par rapport à ce qui se présente réellement au regard de l'opérateur en vision oculaire 3D.

Ce geste médical n'est pas sécurisé : nous pouvons affirmer qu'aucune solution simple et surtout sécurisée n'a été trouvée pour répondre au besoin du clinicien. Pour que son geste soit sécurisé, il doit voir sa zone qui a été radiographiée et la zone où il travaille confondues en temps réel dans un même référentiel. Ceci est la condition indispensable pour pouvoir travailler en toute sécurité, de manière rapide, dans un total confort et avec la précision exigée pour ce type d'intervention.

US 6,006,126 A divulgue un dispositif de visualisation comprenant un émetteur de rayons pénétrants adapté à prendre une vue d'une partie interne située sous une surface externe d'un organe, une paire de lunettes à réalité augmentée ayant, d'une part, un verre optique au travers duquel un utilisateur de la paire de lunettes peut voir et, d'autre part, une caméra de visualisation adaptée à prendre en image ce que l'utilisateur voit au travers du verre optique, une unité centrale étant adaptée à corréler des premières images correspondantes à celles prises par la caméra de visualisation avec des secondes images correspondantes à celles prises par l'émetteur de rayons pénétrants, en sorte que soit projetée sur ledit verre optique ladite vue d'une partie interne.

La présente invention a pour but de remédier à ces inconvénients précités en proposant un nouveau dispositif de visualisation.

Selon un premier aspect, l'invention concerne un dispositif de visualisation de l'intérieur d'une bouche d'un patient, le dispositif de visualisation comprenant un émetteur de rayons pénétrants adapté à prendre une vue d'une partie interne située sous une surface externe d'un organe disposé dans la bouche, caractérisé en ce qu'il comprend une paire de lunettes à réalité augmentée ayant, d'une part, un verre optique au travers duquel un utilisateur de la paire de lunettes peut voir l'intérieur de la bouche, et, d'autre part, une caméra de visualisation adaptée à prendre en image ce que l'utilisateur voit au travers du verre optique, une unité centrale étant adaptée à corréler des premières images correspondantes à celles prises par la caméra de visualisation avec des secondes images correspondantes à celles prises par l'émetteur de rayons pénétrants, en sorte que soit projetée sur ledit verre optique une corrélation d'images constituée :

- de ladite vue d'une partie interne,
- de la visualisation directe au travers desdites lunettes et
- de ladite image prise par la caméra,

ladite partie interne étant ainsi visible à travers ledit verre optique, en superposition à la visualisation directe et à ladite image prise par la caméra.

Selon un premier mode de réalisation du premier aspect de l'invention, l'unité centrale est adaptée à orienter les secondes images en fonction de l'orientation de la paire de lunettes à réalité augmentée.

Selon un second mode de réalisation du premier aspect de l'invention, l'unité centrale est adaptée à projeter sur le verre optique la corrélation des premières images avec les secondes images.

Selon une particularité du second mode de réalisation du premier aspect de l'invention, l'unité centrale est adaptée à projeter sur le verre optique, sur commande de l'utilisateur, des images d'une sélection de composants anatomiques de l'organe pris par l'émetteur de rayons pénétrants.

Selon un troisième mode de réalisation du premier aspect de l'invention, le dispositif de visualisation comprend une caméra de prise d'empreinte optique adaptée à prendre une empreinte optique d'une surface externe d'un organe disposé dans la bouche, l'unité centrale étant adaptée à corréler des troisièmes images correspondantes à celles prises par la caméra de prise d'empreinte optique avec les premières images.

Selon un quatrième mode de réalisation du premier aspect de l'invention, la corrélation des images réalisée par l'unité centrale est une superposition des images sur le verre optique.

Selon un cinquième mode de réalisation du premier aspect de l'invention, l'unité centrale est adaptée, sur commande de l'utilisateur, à modifier le contraste et la transparence des images qu'il traite.

Selon un sixième mode de réalisation du premier aspect de l'invention, l'émetteur de rayons pénétrants est adapté à transmettre numériquement à l'unité centrale les images qu'il prend.

Selon un septième mode de réalisation du premier aspect de l'invention, le dispositif de visualisation comprend un dispositif de numérisation adapté à numériser les images non numériques émises par l'émetteur de rayons pénétrants et à transmettre les images numérisées à l'unité centrale.

Selon un huitième mode de réalisation du premier aspect de l'invention, l'unité centrale est adaptée à projeter sur le verre optique des informations supplémentaires relatives au patient.

Selon une première particularité du huitième mode de réalisation du premier aspect de l'invention, les informations supplémentaires relatives au patient comprennent des données à respecter pour la réalisation d'une prothèse dentaire.

Selon une deuxième particularité du huitième mode de réalisation du premier aspect de l'invention, le dispositif de visualisation comprend au moins un instrument périphérique relié à l'unité centrale et adapté à capter des informations supplémentaires relatives au patient.

Selon un mode avantageux de la deuxième particularité du huitième mode de réalisation du premier aspect de l'invention, l'un des instruments périphérique permet soit de capter l'occlusion statique et les mouvements mandibulaires, soit de capter la couleur des dents, soit de capter la forme du visage, soit de capter les données physiologiques du patient.

Selon un neuvième mode de réalisation du premier aspect de l'invention, le dispositif de visualisation comprend un microphone adapté à capter des ordres de commande provenant de l'utilisateur et à les transmettre à l'unité centrale.

Selon un dixième mode de réalisation du premier aspect de l'invention, la paire de lunettes à réalité augmentée comprend un instrument de repérage spatial.

Selon un onzième mode de réalisation du premier aspect de l'invention, le dispositif de visualisation comprend un système d'éclairage adapté à éclairer l'organe disposé dans la bouche.

Selon une particularité du onzième mode de réalisation du premier aspect de l'invention, le système d'éclairage comprend des diodes électroluminescentes dont la longueur d'onde est adaptée à permettre le repérage de pathologies.

Selon un douzième mode de réalisation du premier aspect de l'invention, l'unité centrale est adaptée à projeter sur un écran déporté des images relatives à l'organe disposé dans la bouche.

Selon un second aspect, l'invention concerne un ensemble de suivi médical comprenant un dispositif de visualisation conforme au premier aspect de l'invention et un instrument de traitement médical qui comporte, d'une part, un outil adapté à traiter des composants anatomiques d'un organe avec lesquels il est en contact, et, d'autre part, un repère adapté à être repéré spatialement lors du traitement des composants anatomiques, l'unité centrale étant adaptée à connaître les dimensions de l'outil et la distance séparant l'outil du repère, et à déterminer la position de l'outil dans l'organe lors du traitement.

Selon un premier mode de réalisation du second aspect de l'invention, l'unité centrale est adaptée à réaliser des quatrièmes images qui représentent l'outil utilisé pour le traitement, à les corréler avec les secondes images, et à projeter la corrélation de façon à permettre la visualisation de l'outil dans l'organe en cours de traitement.

Selon un second mode de réalisation du second aspect de l'invention, la longueur du déplacement de l'outil étant égale à la longueur du déplacement du repère, l'unité centrale est adaptée à déterminer la direction et le sens du déplacement de l'outil par rapport aux composants anatomiques avec lesquels il est en contact, la direction et le sens du déplacement de l'outil étant soit égaux à la direction et au sens du déplacement du repère si l'outil est indéformable par rapport à ces composants anatomiques, soit déterminés par le relief de ces composants anatomiques si l'outil est déformable par rapport à ces derniers.

Selon un troisième mode de réalisation du second aspect de l'invention, l'unité centrale est adaptée à déterminer le mouvement idéal de l'outil utilisé pour la réalisation d'un traitement.

Selon une particularité du troisième mode de réalisation du second aspect de l'invention, l'unité centrale est adaptée à guider l'utilisateur pour que l'outil utilisé suive le mouvement idéal.

Selon un premier mode avantageux de la particularité du troisième mode de réalisation du second aspect de l'invention, le guidage de l'utilisateur est réalisé par l'affichage du mouvement idéal sur le verre optique corrélé avec les secondes images.

Selon un second mode avantageux de la particularité du troisième mode de réalisation du second aspect de l'invention, le guidage de l'utilisateur est réalisé par l'émission d'un signal sonore dépendant de la position de l'outil utilisé.

Selon un quatrième mode de réalisation du second aspect de l'invention, l'unité centrale est adaptée à recevoir un identifiant propre à chaque outil pouvant être utilisé et à déterminer l'outil utilisé.

Selon une particularité du quatrième mode de réalisation du second aspect de l'invention, l'unité centrale comprend une bibliothèque associant à chaque outil pouvant être utilisé l'identifiant qui lui est propre.

Selon un troisième aspect, l'invention concerne un ensemble de réalisation de prothèses comprenant un dispositif de visualisation conforme au premier aspect de l'invention et une machine à commande numérique commandée par l'unité centrale pour la réalisation d'une prothèse relative à l'organe disposé dans la bouche.

Ainsi, l'invention permet de réunir dans un même champ, parfaitement corrélé ou très proche, la visualisation directe au travers de lunettes à réalité augmentées de la zone opératoire que voit le praticien dans la bouche ou sur le visage du patient au travers de ses lunettes, la modélisation obtenue par radiographie (RX, ultrason, IRM ou interférométrie holographique - OCT), additionnée éventuellement de la modélisation issue du traitement découlant de la lecture en empreinte optique d'une caméra endobuccale très précise et de toutes les informations complémentaires pouvant aider le geste opératoire elles même corrélées dans le même référentiel.

Les informations complémentaires peuvent être, par exemple, le chemin suivi par un instrument pour un traitement canalaire ou chirurgical ou par un foret en implantologie, l'instrument et le foret étant normalement invisibles sans utilisation de rayons X. Il est ainsi possible de suivre en temps réel dans la bouche du patient, sans augmentation des expositions aux rayons X, des gestes invisibles au travers de lunettes normales.

Cette invention répond donc totalement aux problèmes exposés en proposant une solution modulable, peu onéreuse et utilisable dans tous les cabinets dentaires sous une forme simplifiée et agréable pour le patient.

En particulier elle répond aux nombreux problèmes évoqués précédemment :

- Par cette organisation nouvelle et originale le praticien peut voir au travers de ses lunettes à réalité augmentée, dans le même champ c'est-à-dire dans la bouche de son patient, (a) la partie du corps qu'il analyse et sur laquelle il travaille, (b) la vue sous-gingivale et osseuse obtenue à partir des appareils de radiologies, ultrason, IRM ou interférométrie holographique (OCT), (c) éventuellement s'il souhaite de la précision, la modélisation qu'il obtient par empreinte optique avec sa caméra endobuccale de lecture tridimensionnelle, les trois vues étant totalement confondues sans l'aide de l'écran déporté. En effet si le praticien souhaite suivre l'évolution de son travail de chirurgie (implantologie, extractions ...) ou d'endodontie, il verra par surimpression ou toute autre forme visualisable comme une variation d'intensité, de couleur ou de contraste, et cela n'est donné qu'à titre d'exemple, la surface sus-gingivale (dents et gencives ...) et la partie sous-gingivale (os, nerfs, vaisseaux, sinus ...) sans quitter la vue de la zone du corps sur laquelle il travaille et fait son diagnostic. Il pourra donc contrôler en temps réel ou différé l'environnement et le résultat de son acte sus et sous-gingival sans détourner le regard de son champ opératoire.
- Par la correspondance de ces informations, il ne risque plus de faire des mouvements de ses mains néfastes et incontrôlés durant son travail, avantage d'autant plus important s'il veut suivre en permanence ses actions dans les zones inaccessibles au regard, sans avoir recours à des rayonnements pénétrants (RX ...) .
- Par la suppression du détournement de son regard de son champ opératoire il ne risquera plus de provoquer une blessure dans la bouche ou sur le corps du patient car ses gestes et les informations attachés au résultat de son action ou l'aidant pour leurs réalisations car ils seront en permanence visibles dans sa zone de travail.
- Par le choix de faire une corrélation entre la vue réelle et la vue invisible sous gingivale et osseuse après traitement de l'information, il est possible d'utiliser tout type de méthode de prise d'empreinte optique précise que ce soit une empreinte issue de méthode utilisant une lumière active structurée ou non. Il est possible aussi d'utiliser tout type de rayonnements pénétrants comme les rayons X, les ultrasons, l'IRM ou l'interférométrie holographique (OCT). Cette méthode de surimposition et/ou substitution en réalité augmentée est totalement indépendante du type de lecture adoptée comme le sont les informations complémentaires issues de la réalité augmentée.
- Par l'utilisation d'une unité centrale, il pourra mémoriser le suivi de tous ces actes, ce qui est très important lors d'expertises (implantologie, sémiologie temporelle ou postopératoire ...) .
- Par l'absence de tous mouvements des yeux risquant d'impliquer une forte gymnastique oculaire à un rythme très élevé, l'opération deviendra très reposante pour le clinicien.
- Par l'utilisation de lunettes disposant de la possibilité d'afficher une réalité augmentée il sera possible de donner des informations en temps réel ou différé, au choix du clinicien, dans le champ opératoire. Cela inclut toute information complémentaire directement corrélée sur le champ visualisé comme le permet aujourd'hui la réalité augmentée mais aussi des informations issues de source d'informations complémentaires comme celles issues de la télémédecine.
- Grâce aux informations complémentaires optionnelles de la réalité augmentée, cela permet aussi :

- De guider l'opérateur sur site par télémédecine mais aussi par système expert ou apprentissage personnalisé si des zones importantes ne sont pas traitées correctement.
- De montrer spécifiquement et sur site des informations sous-gingivales d'environnement fragile ou importantes.
- De mettre en garde le clinicien durant l'acte opératoire si celui-ci n'est pas parfaitement exécuté. Il est possible par exemple d'indiquer des traitements radiculaires incomplets, des forages de fût d'implant insuffisamment ou mal positionnés, d'extractions ou de curetages incomplets.
- De faire apparaitre et de permettre de visualiser sur site les mouvements dynamiques des instruments utilisés ou des parties du corps en traitement lors de réalisation d'extractions difficiles, de la pose d'implants ou de forages de canaux radiculaires.
- De mettre en évidence en bouche la distribution des tissus dentaires, par exemple la proximité de la pulpe, durant la préparation de cavités destinées à recevoir une obturation et une couronne.
- De suivre en bouche et en temps réel le chemin suivi par tout instrument qu'utilise le clinicien afin d'augmenter son efficacité et d'éviter des accidents sur l'environnement (veines, nerfs ...).

- Par les moyens mis en oeuvre, le dispositif est simple dans sa fabrication ce qui le rend particulièrement résistant. Cela permet aussi :

 - De réduire significativement le prix de fabrication, donc le prix de vente depuis la démocratisation des éléments électroniques utilisés (caméras de nouvelles générations Condor, lunettes à réalité virtuelles, LEDs).
 - De choisir une connexion câblée ou non câblée, y compris au niveau de la caméra, libérant complètement les gestes du clinicien.
 - D'avoir la restitution 3D naturelle stéréoscopique sans être obligé d'utiliser des écrans 3D toujours chers et souvent peu performant.

D'autres buts et avantages de la présente invention apparaitront dans la description qui va suivre, se rapportant à un mode de réalisation donné à titre d'exemple indicatif et non limitatif. La compréhension de cette description sera facilitée au vu des dessins joints en annexe et dans lesquels :

- La figure 1 est une représentation schématique de l'ensemble du dispositif comprenant l'ensemble des éléments principaux indispensables à son bon fonctionnement mais aussi les éléments périphériques complémentaires mais non obligatoires ;
- La figure 2 est une représentation d'ensemble du prototype en parti réalisé comprenant la caméra, la connectique, l'ordinateur (ici un portable) et éventuellement un boîtier renfermant les cartes de traitement ;

- La figure 3 représente un diagramme complet des éléments essentiels du dispositif propre à l'invention ;
- La figure 4 représente les différentes étapes de corrélation entre la partie visible et invisible permettant la création de l'objet complémentarisé basées sur leurs parties communes, ici les couronnes des dents ;
- La figure 5 représente les différentes vues d'un objet complémentarisé observées par le clinicien dans la bouche de son patient au travers des lunettes à réalité augmentée lorsqu'il déplace son regard ;
- La figure 6 représente les différents plans observables par le clinicien en bouche de l'objet complémentarisé lorsqu'il fait appel à la fonction transparence de la présente invention ;
- La figure 7 représente la vue de l'objet complémentarisé dans l'application de la présente invention lors de la réalisation de préparations prothétiques ;
- La figure 8 représente la vue de l'objet complémentarisé observé en bouche par le praticien lorsqu'il utilise un instrument mémorisé ou reconnaissable et déformable pour un traitement canalaire ou indéformable pour le forage d'un implant ou une opération chirurgicale ; et
- La figure 9 est un diagramme représentant les différentes étapes de la manipulation clinique permettant la mise en oeuvre de la présente invention.

La présente invention se rapporte à un nouveau dispositif dans le domaine dentaire de visualisation et/ou de mesure endobuccale directement sur le site de travail c'est-à-dire dans la bouche du patient, réunissant dans un même référentiel tridimensionnel ou légèrement décalé, (a) la vue directe des dents et gencives dans la bouche du patient à travers les lunettes à réalité augmentée, (b) une ou plusieurs modélisation issues de prises d'empreintes radiologiques, OCT et/ou IRM, (c) une ou plusieurs références ou modélisations issues des caméras présentent sur les lunettes à réalité augmentée au travers desquelles le praticien visualise la bouche du patient, (d) éventuellement une ou plusieurs références ou modélisations issues d'une empreinte optique utilisant ou n'utilisant pas de lumière structurée faite à l'aide d'une caméra endobuccale, afin qu'elles se complètent et/ou se substituent mutuellement pour s'enrichir en utilisant le principe de la réalité augmentée, et (e) éventuellement des informations complémentaires associées aux présentes fournies par d'autres périphériques, dans le but de permettre au clinicien de ne jamais détourner son regard de son site de travail endobuccale, notamment lors de traitements gingivaux, coronaires, radiculaires, chirurgicaux ou osseux afin de sécuriser, faciliter et optimiser son acte clinique, cette application n'étant toutefois pas limitative, en ce sens que le dispositif est aussi applicable dans le suivi de leurs ensemble, des activités cliniques du cabinet dentaire.

Pour ce faire, ce dispositif selon l'invention permet de visualiser directement dans la bouche du patient, au travers de lunettes à réalité augmentées, et de manière parfaitement corrélée à la fois la partie visible et la partie invisible de la bouche sous la forme d'un seul objet que l'on nomme ici l'objet complémentarisé.

La partie visible est la surface des dents de la gencive, de la langue et de l'intérieur des joues. Elle peut être vue directement au travers des lunettes mais elle peut être aussi vue sous la forme d'une vue numérisée issue de la lecture d'une caméra par stéréodynamique, ou de plusieurs caméras situées sur les lunettes ou, de manière plus précise, grâce à la numérisation obtenue par le scannage effectué à l'aide de la caméra endobuccale. La numérisation peut se substituer sans que cela soit obligatoire, à la vision directe dans les parties numérisées de la moins précise à la plus précise, c'est-à-dire par ordre de domination la plus précise (la caméra endobuccale) se substitue à la moins précise (caméra des lunettes) qui elle-même peut se substituer à la vue direct non numérisée.

La partie invisible est issue d'une lecture effectuée séparément avant l'action thérapeutique grâce à des appareils de type périphérique capable de fournir des images RX, IRM, térahertz ou ultrasoniques des parties invisibles qui se situent sous les dents, sous la gencive ou sous la peau, comme le sont l'os, les tissus épithéliaux et conjonctifs, les vaisseaux et les nerfs. Ces appareils permettant de connaître, mémoriser et numériser l'anatomie sous-jacente invisible de manière statique ou dynamique.

Pour que ces deux ensembles volumiques, la partie visible et invisible ne forme plus qu'un seul ensemble, l'unité centrale cherche les parties communes et réunit les deux objets en s'appuyant sur ces parties communes. Ces parties communes peuvent être des objets anatomiques comme les couronnes des dents ou des objets rapportés comme le sont des cales de repérage fixées dans la bouche, par exemple sur les dents si l'on souhaite éviter toute mobilité anormale. Ces cales ou ces repères anatomiques servent aussi de repérage pour suivre le mouvement des instruments réalisés à cet effet.

Préférentiellement et dans certain cas il est possible de faire la numérisation en même temps des partie invisibles et visibles (notamment dans le cas d'un appareil ultrasonique ou de térahertz) .

Pour ce faire, l'invention se compose 1) d'un dispositif de visualisation travaillant en temps réel ou différé utilisant des lunettes à réalité augmentée auxquelles peut-être associé un système de repérage spatial tridimensionnel (accéléromètres/gyroscope et une caméras aux minimum) et ayant pour fonction de permettre au praticien, non seulement de voir son champ opératoire en vision directe, mais aussi d'avoir des indications ponctuelles ou des vues externes comme le font toutes les lunettes de ce type pour l'assistance à la chirurgie, ce qui lui permet de suivre normalement la progression normalement visible de son travail (par exemple de traitement endocanalaire ou des actions chirurgicales) c'est-à-dire la partie externe des dents et des gencives. Elle lui permet aussi de surajouter des images corrélées, et ceci est la caractéristique essentielle de l'invention, issues 2) d'un deuxième périphérique. Ce deuxième périphérique est capable de fournir des images RX, IRM, térahertz ou ultrasoniques des parties invisibles qui se situent sous les dents et sous la gencive comme les sont l'os, les tissus épithéliaux et conjonctifs, les vaisseaux et les nerfs, et de permettre de connaître, mémoriser et numériser l'anatomie sous-jacente invisible. Ces deux périphériques 1) et 2) sont sous la dépendance 3) d'une unité centrale ayant pour fonction de numériser les vues issues des caméras situées sur les lunettes et sur le périphérique dans le but de les corréler pour les réunir dans le même référentiel afin que le clinicien ne voit dans la bouche de son patient, au travers de ses lunettes à réalité augmentée, qu'un seul objet issu de la fusion de la vue qu'il a naturellement au travers de ses lunettes à laquelle sont fusionnées en permanence, de manière dynamique et en temps réel ou quasi réel, les différentes informations provenant à la fois des éléments externes des dents et de la gencive mais aussi des éléments invisibles ce qui permet à ce dernier d'avoir dans son champ de vision dans la bouche de son patient la partie visible mais aussi la partie invisible se situant sous la gencive et sous les dents.

Cela permet donc à l'utilisateur de suivre son action sans détourner le regard et de connaître la conséquence de son acte dans une partie normalement inaccessible à son regard dans la bouche. Il est à noter que le présent dispositif, pour éviter une irradiation permanente du patient n'a besoin au minimum que d'une image 3D initiale et qu'il la corrélera en temps réel ou quasi réel en fonction de champ de vision, variable suivant l'orientation du regard du praticien, que filment les caméras présentes sur les lunettes à réalité augmentée.

A ce dispositif, et préférentiellement, sera adjoint 4) une caméra endobuccale de prise d'empreinte optique précise utilisant des rayonnements cohérents ou non, avec projection ou sans projection de lumière active et/ou structurée ayant pour fonction de faire un relevé très précis des formes et couleurs de l'anatomie présentes dans la partie visible de la bouche du patient comme ses dents et/ou sa gencive, cette prise d'empreinte étant corrélée et fusionnée par l'unité centrale 3) aux vues précédentes et plus particulièrement à la vue moins précise issue des caméras portées par les lunettes à réalité augmentée 1) mais aussi et de ce fait aux images sous épithéliales issue du périphérique externe 2). Ceci permet au clinicien d'avoir dans son champ de travail une vue extrêmement précise de la partie issue du traitement à réalité augmentée.

Eventuellement et préférentiellement sera adjoint 5) un système d'éclairage sur la caméra endobuccale ou sur les lunettes ayant pour fonction d'optimiser l'analyse diagnostic comme par exemple la mise en évidence par des rayonnements spéciaux des zones carieuses sur les tissus durs ou des tumeurs sur les tissus mous.

Pour suivre les mouvements en endodontie ou en chirurgie, il suffit de corréler des instruments visualisés, connus ou calibrés sur la double vue visible et invisible.

Ce procédé permet d'optimiser l'action thérapeutique du clinicien en augmentant significativement la sécurité nécessaire à ses actes tout en assurant l'intégrité structurale du corps humain et en offrant une précision de l'ordre du micron. Il permet surtout de totalement libérer le praticien de certaines contraintes l'obligeant à regarder sur un écran déporté, à corréler des images différentes d'une zone visible et invisible et à rester à proximité de son unité de travail.

L'invention comprend un dispositif de matériel informatique et un système logiciel.

Le dispositif de matériel informatique associe 1) un système de visualisation dentaire direct des tissus visibles associés à de la réalité augmentée spécifique et miniaturisé, 2) un système de numérisation des tissus sous-jacents invisibles à l'aeil, 3) une unité centrale de conversion analogiques/digitales, de gestion et de corrélation des données, 4) éventuellement une caméra de lecture tridimensionnelle endobuccale précise avec ou sans lumière structurée, 5) éventuellement un éclairage endobuccale spécifique aux diagnostic, et 6) des instruments calibrés et connus utilisés dans les champs visibles et invisibles.

A cet effet la présente invention a pour objet un dispositif comprenant plus précisément un système de visualisation/captage avec des lunettes à réalité augmentée, un dispositif permettant de numériser les parties invisibles à l'œil, une unité centrale, une caméra de numérisation précise endobuccale, un système d'éclairage et des périphériques accessoires.

Le système de visualisation/captage avec des lunettes à réalité augmentée 1 permet de voir la zone d'action thérapeutique en vision directe tout en pouvant y corréler puis y ajouter, si elles ont des zones communes de raccordement, des informations complémentaires invisibles à l'œil directement issue de périphériques indépendants comme par exemple les images issues de systèmes de lecture RX, IRM, térahertz ou ultrasonique.

Ce système de visualisation, d'affichage et de captage peut être constitué, par exemple, des lunettes « Google Glass », « Vuzix smart Glass », « sony », « K-Glass » ou « HoloLens ».

A ces lunettes est adjoint une ou plusieurs caméras permettant de recaler en permanence et en temps réel, par des alignements ou mises en correspondance successifs, la modélisation issue de la lecture des périphériques sous-gingivaux en utilisant des repères communs comme le sont, par exemple, les couronnes des dents ou des repères volontairement placés sur leurs surfaces ou sur la gencive sur ce que voit le clinicien dans la bouche du patient.

Eventuellement, avantageusement selon une caractéristique additionnelle du dispositif selon l'invention, pour des raisons financières, le dispositif peut s'appuyer sur une visualisation 2D, les lunettes ont pour fonction essentielle de faire apparaitre des informations complémentaires avec un ajustement imprécis sur la zone de travail vue en relief. L'unité centrale est aujourd'hui en mesure de corréler les vues 2D sur un motif 3D.

Il peut aussi créer une image 3D en utilisant deux ou plus images 2D radiologiques.

Dans le cas d'une visualisation 2D 1/2 ou 3D, c'est-à-dire dans la mesure où ces lunettes disposent d'une vision spatiale, en général utilisant la stéréoscopie sans que ceci soit systématique. La corrélation est très exacte et les indications se font sur des parties du corps lues en trois dimensions. Ceci est rendu possible grâce à la présence d'écrans spécifiques dédiés existant sur ce type de lunettes.

Avantageusement et selon une caractéristique additionnelle du dispositif selon l'invention, la présence d'un mini micro mini-USB sur la branche permet de donner des ordres de vision et d'apparition des informations en réalité augmentées sans que l'opérateur ai à déplacer son regard de sa zone de travail.

Le dispositif permettant de numériser les parties invisibles à l'aeil (l'émetteur de rayon pénétrants 2) peut être un système radiologique analogique (passant alors par une tablette de numérisation) ou numérique 2D ou 2D 1/2 par exemple et ceci n'est pas une limite de l'invention, de type RVG scanner ou tomographie. Il peut aussi utiliser des systèmes d'optique cohérente pénétrant comme l'est par exemple l'OCT. Il peut aussi utiliser les principes d'imagerie 3D de l'IRM ou des béta caméras. Très récemment est apparue l'imagerie térahertz. Celle-ci a l'inconvénient d'être encore peu précise mais elle a un grand avantage d'utiliser un vecteur non ionisant. Elle peut être utilisée comme périphérique, partie de l'invention. Il en est de même de tous les systèmes ultra-soniques qu'elle qu'en soit le type.

Le but de ce deuxième composant propre de l'invention, est de collecter les informations invisibles à l'œil pour créer un deuxième objet permettant de venir compléter l'objet créé lors de la visualisation des parties visibles.

L'unité centrale 3 permet de conversion des données analogiques/digitales et de gestion de ces données. L'avantage de ce système est de numériser les données issues des caméras situées sur les lunettes, de numériser et/ou de recueillir les images issues des appareils périphériques (RX, IRM, OCT, ultrason ...) puis de les réunir pour obtenir un seul nuage de point permettant de constituer un seul objet.

En plus de cette réunion, avantageusement et selon une caractéristique additionnelle du dispositif selon l'invention, l'unité centrale oriente la partie invisible en fonction de l'orientation du regard du clinicien, cette indication étant apportée par les caméras, via le repère, et/ou les systèmes additionnels comme les gyroscopes ou autre appareil permettant de connaître le positionnement d'un objet, ici les lunettes à réalité augmentée, dans l'espace.

Grâce à cette application de notre invention, l'unité centrale peut suivre la variation de la position spatiale du regard ce qui permettra non seulement de voir la partie invisible mais aussi de la visualiser directement dans la bouche du patient sous différents angles de vues. Cette caractéristique est importante car, cliniquement, certaines structures anatomiques peuvent masquer des zones importantes. Le praticien, en déplaçant son regard sera capable de voir ce qui était caché dans l'angle de vue précédent.

Avantageusement et selon une caractéristique additionnelle du dispositif selon l'invention, l'unité centrale pourra faire apparaitre préférentiellement les vaisseaux, les nerfs, l'os, les racines car les logiciels actuels sont capables de discerner automatiquement ces structures anatomiques et les afficher dans différentes couleurs.

Cette distinction permet au praticien de connaître son champ de travail, de le sélectionner mais aussi de s'adapter à une anatomie propre au patient traité. L'invention permet de passer de l'anatomie standard à l'anatomie personnalisée ce qui est tout particulièrement important en implantologie ou en chirurgie dentaire.

Le dentiste voit donc dans la bouche de son patient les dents, la gencive mais aussi toutes les structures sous-jacentes comme les racines des dents, les vaisseaux sanguins, les nerfs..., sous tous les angles et de manière sélective avec éventuellement des couleurs spécifiques.

La caméra de numérisation précise endobuccale 4 permet de numériser une ou plusieurs dents par empreinte optique utilisant des rayonnements photoniques cohérents ou non ou ultrasoniques. L'invention peut utiliser toutes les caméras utilisées dans le monde dentaire et médical, ce qui montre l'ouverture et l'universalité de l'invention.

Cette caméra peut faire ses relevés métriques en utilisant des projections de lumière structurée. Dans ce cas, la caméra possède deux ou plus de deux canaux, confondus ou séparés, l'un de projection et l'autre de reprise d'image. Un système de lumière structurée par LED, OLED, halogène, plasma ou laser projette sur les dents un rayonnement sous la forme de points, de lignes ou de trames connus et structurés. Cette projection structurée est déformée en fonction des surfaces qu'elle frappe et cette déformation est transmise sur un capteur par la voie de reprise d'image. Ceci permet à la caméra, par comparaison entre les caractéristiques de la lumière projetée ou mémorisée et la lumière déformée dans l'espace ou/et le temps qui arrive sur le capteur, de connaître la forme et les dimensions des dents objet de l'analyse. On trouve de nombreuses caméras endobuccales répondant à ces caractéristiques.

Avantageusement et selon une caractéristique additionnelle du dispositif selon l'invention cette caméra peut utiliser tout système de mesure et d'analyse des formes des dents et/ou de la gencive sans projection de lumière structurée. Pour se faire, elle peut utiliser des méthodes télémétriques ou stéréoscopiques mono ou multi-caméra. Ce système à l'avantage d'être plus simple à concevoir mais oblige à développer des logiciels plus complexes tels que ceux développés pour le spatial. Nous y trouvons, quelques caméras endobuccales, par exemple et ceci n'est qu'un exemple non limitatif, celle que nous avons développé sous le nom de Condor.

Avantageusement et selon une caractéristique additionnelle du dispositif selon l'invention, il peut aussi comprendre des caméras associant les deux technologies ou d'autres principes comme l'OCT, l'ultrason ou les rayons X dans la mesure où celles-ci fournissent des informations métriques sur la zone et le corps étudiés.

Certes il est possible d'utiliser un éclairage naturel mais, comme ce type de caméra a pour fonction de travailler dans des zones sombres ou difficilement accessibles (par exemple la bouche), il est possible d'avoir un système d'éclairage 5 permettant une illumination bien dimensionnée de la zone de travail.

Avantageusement et selon une caractéristique additionnelle du dispositif selon l'invention, le système d'éclairage peut faire apparaitre des informations sur les objets mesurés en réalité augmentée et en 3 dimensions en fonction du type d'éclairage utilisé. En effet suivant le choix de certaines longueurs d'onde, il est possible de déterminer et/ou de retrouver certains éléments anatomiques et pathologiques de la sphère oraux-faciale invisible ou peu visible à l' oeil et de les indiquer dans le champ opératoire, sous la forme d'information en réalité augmentée, à la différence des visualisations directes en 2D sur un écran vidéo déporté. Cela permet le diagnostic mais aussi certains éléments de calage permettant la corrélation entre l'image du visible et du sous-jacent pour construire l'objet complémentarisé.

Les périphériques accessoires peuvent être :

- Une source d'informations 6 directement issue des fonctions mémorisées ou de logiciels intra ou extra muros (télémédecine) apportant des informations complémentaires permettant d'aider au geste médical de prise d'empreinte et pendant la préparation.
- Un ou plusieurs postes périphériques 7 où sont visibles les informations avec lesquelles travaille le clinicien et qui peuvent être vues par ses assistants afin qu'ils puissent suivre et enrichir en temps réel ou différé les travaux effectués (assistance ou enseignement..). Ce traitement peut être vidéo et /ou numérique.
- Des instruments endobuccaux calibrés et corrélés à l'image de la partie visible et invisible permettant de suivre les mouvements en temps réel dans la partie invisible.
- Une machine-outil à commande numérique 8 pouvant, à tout moment, réaliser une pièce réelle de l'image virtuelle captée afin que ce dispositif trouve sa totale application dans la chaine de CFAO dentaire.

Sont associés avantageusement et selon une caractéristique additionnelle du dispositif selon l'invention pour transmettre les données issues du dispositif ou de ses périphériques :

- Une transmission de toutes les données par câble, téléphone, Bluetooth ou Wifi.
- Un système de matériel informatique de traitement complémentaire, de dialogue/visualisation avec l'opérateur, les assistants et/ou l'unité centrale, de transmission et de stockage des informations, des ordres et des données comme le permet le micro du système d'affichage ou une autre forme de communication.

En accordance avec le présent montage de matériel informatique est proposée une méthode logicielle répondant aux exigences de rapidité et de précision nécessaires à l'homme de l'art dentaire et permettant de faciliter significativement son geste opératoire.

Le système logiciel original comprend :

- Un schéma de reconstruction 3D temps réel à partir de deux flux d'images 2D provenant des deux caméras ou plus du système de visualisation à réalité augmentée ;
- Un schéma de reconstruction 3D temps réel à partir d'un flux d'images 2D, 2D1/2 ou 3D provenant d'une seule du périphérique RX et autre capable de visualiser les éléments invisibles à l'œil ;

- Un algorithme de recherche de points d'intérêt sur les trois algorithmes de recherche de trace optique (projection sur plusieurs caméras différentes d'un même point 3D) par calcul de points d'intérêt et mise en correspondance à travers les images ;
- Un algorithme de séquençage automatique temps réel du flux d'images en sous séquences spatialement cohérentes permettant de suivre le mouvement du regard du clinicien ;
- Un algorithme d'estimation parallèle des positions des caméras dans l'espace et des coordonnées des points 3D grâce aux traces optiques ;
- Un algorithme d'interpolation 3D de nuages de points ;
- Un algorithme de polygonisation de nuages de points 3D et de calcul de texture ;
- Un algorithme de mise à l'échelle des reconstructions 3D ;
- Deux algorithmes de rehaussement de précision spatiale ;
- Deux algorithmes de sélection des éléments anatomiques tenant compte, entre autre, des variations de contraste et de densité ;
- Un algorithme d'affichage de l'objet complémentarisé enrichi des sélections d'affichage des éléments anatomiques dans l'objet complémentarisé ; et
- Des algorithmes de corrélation des mouvements dynamiques d'instruments connus et utilisés par le praticien.

L'organisation globale de l'algorithme est le suivant :
Le flux d'images, en provenance de la ou des caméras, est traité en temps réel de manière à produire une première reconstruction 3D visualisable par l'utilisateur au fur et à mesure que celui-ci déplace son regard aux alentours de l'objet. Le schéma global de reconstruction 3D temps réel et l'organisation des données varient en fonction de la disponibilité des deux (ou plus) caméras du système à réalité augmentée 1 et du périphérique 2 captant les informations invisibles en temps différé.

Chaque image nouvellement acquise est d'abord traitée par l'algorithme de recherche de trace optique. A partir des correspondances, l'algorithme de séquençage met alors à jour le séquençage du flux vidéo pour une meilleure performance temporelle. L'algorithme d'estimation parallèle permet ensuite, grâce aux traces optiques des périphériques émettant des rayons pénétrants 2 (RX, ultrason, IRM ...), a) de retrouver les positions des caméras dans l'espace au moment de l'acquisition et b) de générer le nuage de points 3D se projetant sur les traces optiques des caméras des lunettes et des périphériques.

Le nuage unique de points généré est ensuite interpolé (algorithme) pour obtenir un nuage plus dense, et une fonction implicite d'interpolation est calculée. Grâce à cette fonction, on obtient une polygonisation texturée de la surface à reconstruire (algorithme). A cette étape, il est aussi possible de calculer des indices de qualité du nuage de points final. Certains points (zones) peuvent ainsi être étiqueté(e)s comme non valides ou comme particulier(e)s (os, vaisseaux, os, racines ...).

La surface texturée est enfin affichée à l'écran sur les lunettes à réalité augmentée, en correspondance avec la vue directe, avec éventuellement des annotations adaptées pour indiquer les zones encore particulières sélectionnées à priori par le clinicien.

La surface générée en temps réel est une représentation sans dimension spatiale représentant à un facteur d'échelle près de la zone reconstruite. Ce facteur d'échelle peut être calculé par l'algorithme en calcul caché temps quasi réel ou en temps réel ou différé lorsque l'acquisition est terminée.

Enfin, le modèle 3D final peut voir sa précision rehaussée par l'algorithme, de manière à avoir une reconstruction la plus fidèle possible. Cet algorithme recalcule un nuage de points 3D en tenant compte de l'ensemble des vues acquises. Ce nuage est ensuite interpolé par l'algorithme. Enfin, un algorithme de modélisation surfacique et/ou volumique vient reconstruire le modèle 3D global affiché.

Nous savons aussi que les images radiologiques sont généralement porteuses d'informations en nuages de points 3D rapportés à des unités élémentaires, les voxels, directement corrélables au nuage de points obtenu au niveau de la vue précise faite par la caméra endobuccale. Par contre il est impossible de fusionner les vues radiologiques directement en bouche sur les vues d'empreintes optiques. L'opérateur doit suivre sur un écran déporté l'environnement anatomique sous-cutané dans lequel il travail et reporter intellectuellement cette vue dans l'espace de son champ opératoire. Ceci conduit très souvent à des erreurs d'appréciations, surtout si l'on admet le phénomène de flèche, c'est-à-dire qu'une imprécision de quelque degré sur un axe d'insertion d'un foret en implantologie ou prothèse, lime d'alésage de canaux dentaires ou d'un trocart en médecine, se traduira par une erreur de plusieurs millimètres à un centimètre de profondeur dans l'os. Le risque de lésion d'organe du corps humain, comme des nerfs, des artères et des veines est donc important.

Avantageusement et selon une caractéristique additionnelle du dispositif selon l'invention, il est possible de faire une triple fusion réalisée au niveau de l'unité centrale 3 : celle de l'empreinte optique précise obtenue à l'aide de la caméra endobuccale 4, celle obtenue au niveau de l'analyse radiologique 2 qu'elle soit en 2D, 2D 1/2 ou 3D, et celle observée par les caméras, au travers des lunettes en réalité augmentée, certes moins précise, mais servant de support aux deux précédentes.

Le dispositif, selon l'invention, permet donc au clinicien de voir, sans avoir à détourner son regard, non seulement une modélisation surfacique précise comme tout système connu d'empreinte optique, mais en plus, une modélisation de ce qui est invisible dans son champ opératoire, c'est-à-dire la partie sous épithéliale et osseuse, fusionnées avec la partie externe. Il n'a donc sous ses yeux qu'un seul champ opératoire où sont visible les parties externes et les parties internes normalement invisibles.

Avantageusement, il est possible de suivre les mouvements des instruments de chirurgie dentaire aussi bien dans les racines (endodontie) que dans l'os (chirurgie et implantologie) assurant une maitrise des actes jusqu'alors impossible en temps réel.

Ainsi il est possible de pratiquer des explorations et des traitements radiculaires ou osseux en suivant le mouvement de l'instrument de travail dans les parties invisibles dans la mesure où celui-ci a été calibré dans le référentiel de la prise d'empreinte optique et/ou radiologique. Le praticien voit au travers de ses lunettes à réalité augmentée 1 l'extérieur de la couronne reportée par la vue visible (voire même améliorée par l'usage d'une caméra de prise d'empreinte optique 4) fusionnée à la vue générale et au travers des lunettes à réalité augmentée 1 et augmentée de la vue invisible de la racine (longueur et forme) directement issue de l'émetteur de rayons pénétrants 2 (périphérique de prise de vue RX, IRM ou terahertz) mais aussi, ce qui est fondamental, avantageux, et selon une caractéristique additionnelle du dispositif, le mouvement de ses instruments de travail à l'intérieur de cette racine ou de l'os (en chirurgie et implantologie).

Les figure représentent différentes mises en oeuvre du dispositif en montrant toutes les possibilités qu'il offre dans la pratique quotidienne du chirurgien-dentiste : les lunettes à réalité augmentée et les caméras de visualisation peu précises 1, les périphériques visualisant les parties invisibles 2, l'unité centrale 3 digitalisant et corrélant les deux vues visibles et invisibles, la caméra endobuccale très précise 4 pour les vues visibles, et l'éclairage spécifique 5.

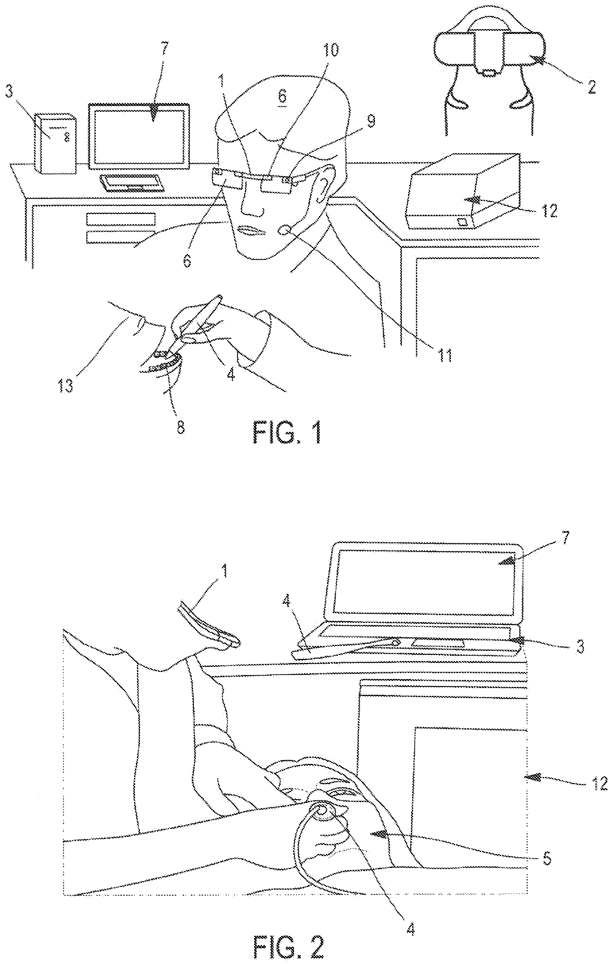

La figure 1 est une représentation de l'invention, sous la forme d'un dessin didactique, faisant apparaitre les éléments essentiels et accessoires pouvant être mis en oeuvre dans ce dispositif de visualisation enrichie réunissant en une seule vue les parties visibles et invisibles grâce au procédé de réalité augmentée et permettant à l'opérateur de ne jamais quitter son champ opératoire lorsqu'il réalise ses mesures et/ou ses diagnostics et/ou ses actes cliniques, dispositif trouvant un intérêt tout particulier dans les domaines de la dentisterie.

Le dispositif comprend des lunettes à réalité augmentées 1, comme par exemple les « Google glass » mais ceci n'est pas limitatif car il existe d'autres lunettes de ce type, le praticien 6 a une vision naturelle stéréoscopique de la bouche, visible sur l'écran 7 donc de la zone qu'il mesure et qu'il étudie 8. Lorsque l'opérateur regarde cette zone de travail, la (les) caméra(s) stéréoscopique(s) 9 faisant partie des lunettes, observe(nt) la même scène et est (sont) capable (s) de procéder à un relevé d'information conduisant à la création d'un nuage de points dit de visualisation. Comme la tête du dentiste peut bouger par rapport à la zone observée, il a été adjoint des accéléromètre/gyromètre/magnétomètre 3D 10 proche des yeux facilitant le suivi dans l'espace l'axe d'observation du clinicien. Ceci n'est pas obligatoire car le logiciel peut utiliser les zones de raccordement mais facilite grandement le travail de l'unité centrale qui fait la corrélation en dynamique de la partie visible et invisible (ci-après appelée complémentarisée) lorsque l'observateur doit déplacer son regard en dehors de la zone de travail, et y revenir pour continuer son travail.

Cette corrélation dynamique fait que, quel que soit l'angle de vision, le clinicien voit les deux parties sous différent angles, ce qui peut être fondamental si dans la partie invisible, une structure anatomique, par exemple une racine de dent, cache une pathologie ou une zone à travailler.

Le fichier invisible de la bouche est apporté par le système d'imagerie périphérique 2. Celui-ci peut être un scanner ou des systèmes de tomographie offrant, par l'assemblage de leurs sections, une vue 2D 1/2 laissant préférentiellement apparaitre les structures osseuses. Pour avoir une vue plus complète ont été adjoint des logiciels très puissants permettant de distinguer les tissus mous sur des images radiologiques avec peu de déformations. Ceci était nécessaire en implantologie où le geste doit être précis si l'on ne veut pas risquer de léser un élément anatomique comme les nerfs ou les vaisseaux sanguins. Le cone beam se trouve dans cette catégorie, il est de plus en plus utilisé car il donne des indications suffisantes sur les tissus durs et les tissus mous invisibles sans trop déformer la vue 2D 1/2 fournie après la reconstruction logicielle. Il est possible d'avoir des informations plus précises directement en 3D dans la mise en œuvre de la présente invention en ayant recours à une technique d'imagerie plus complexe et plus coûteuse comme l'IRM ou les bêtacaméras. Enfin, il est possible d'utiliser comme périphérique émettant des rayons pénétrants 2 des techniques plus récentes comme l'OCT (Optique cohérente tomographie) ou l'imagerie par térahertz qui l'avantage de ne pas être, en commun avec l'IRM, ionisante. Enfin reste l'imagerie par ultrason qui peut permettre de visualiser les tissus sous-jacents en temps réel comme décrit dans le brevet n° FR 83.07840 du 4 mai 1983 « procédé de saisie de la forme d'organes humains ou d'anomalies pathologiques et dispositif pour sa mise en œuvre ». Même s'il ne peut être écarté de la présente invention, le problème de l'ultrason reste son imprécision.

Dans tous les cas, les périphériques actuels 2 permettent de numériser la partie invisible de la bouche et de séparer les différents composants anatomiques dans le but de les faire apparaître ou disparaître spécifiquement, car ces techniques savent aujourd'hui distinguer la veine de l'artère, les vaisseaux des nerfs, les racines (très denses) de l'os ou le canal radiculaire du reste de la racine. Ceci sera très important dans la manipulation clinique, propre à cette invention, que nous allons décrire plus loin.

La troisième partie du présent dispositif est l'unité centrale 3 en charge de gérer les informations numériques de la surface des parties visibles transmises par les caméras des lunettes à réalité augmentées et celles des parties invisibles transmises en temps réel (par exemple ultrason) ou différé (par exemple cone beam). L'unité centrale permet de trouver les zones communes afin de corréler les deux nuages de points conduisant à la construction d'un seul objet complémentarisé (réunissant visible et invisible en un nuage de point unique). Il s'agit de reporter à tout instant la vue invisible sur la vue visible en s'appuyant sur des éléments communs. Il s'agit aussi de rendre dominant cette partie invisible sur la partie visible avec un indice de transparence réglable.

La présente invention comprend accessoirement une caméra de prise d'empreinte optique 4 permettant au dentiste 6 ou au médecin d'effectuer ses mesures 3D dans la bouche ou sur la peau de son patient avec une grande précision. Cette mesure étant très précise (quelques microns) et très proche des dents, la profondeur de champs étant très faible, ce qui explique qu'il doit procéder à un balayage de l'ensemble des dents 8, par photo successive (one shoot impression) ou par filmage 3D (full motion).

Dans ce cas, les deux mesures, celle obtenue avec la caméra endobuccale 4 et celle obtenue avec les caméras des lunettes disposant de la réalité augmentée 1 fournissent deux fichiers correspondant à la même zone mais n'ayant pas la même précision. Ces fichiers peuvent être de simples informations électro-optiques ou des informations plus sophistiquées comme des représentations numériques sous forme de nuages de points ou même de modélisations surfaciques ou volumiques. Dans tous les cas existent entre ces deux fichiers des valeurs communes, utilisée aussi pour obtenir l'objet complémentarisé, comme par exemple les points se situant dans des zones identifiables facilement comme le sommet des cuspides des dents 8 ou le fond de leurs sillons. Ces valeurs de références communes permettent à l'unité centrale 3 de fusionner les deux fichiers en un seul tout en leur préservant leurs spécificités.

De même l'utilisation d'un éclairage spécifique 5 peut faciliter la lecture 3D des dents qui ont une réflexion très spéculaire. Ainsi la lumière spécifique peut être une projection active et structurée comme par exemple la projection de grilles ou autres motifs. Il est aussi possible utiliser des caméras n'utilisant pas de lumière structurée mais s'appuyant sur les principes de la stéréoscopie passive (AWS ou autre), ou sur la technique comme le temps de vol (ou « time flight ») ou des techniques holographiques ou ses dérivés comme l'OCT. Ce nouveau dispositif est totalement universel et applicable à toute forme de visualisation et/ou de mesures localisées endobuccales. A la différence des techniques architecturale utilisées classiquement par des lunettes à réalité augmentée qui recherchent des points spécifiques, il utilise une double empreinte optique, celle issue des caméras endobuccales 4 et celle réalisées en même temps ou en temps différé au travers des lunettes à réalité augmentée 1 pour les enrichit et/ou les substituer en fonction de leur degré de précision.

De même il est possible d'exporter ces données pour les visualiser sur un écran périphérique 7 pour ses assistants avec lesquels il communique à l'aide d'un micro sur les lunettes ou indépendant 11 ou encore de les exploiter pour faire un usinage 12 de guides implantaires ou parties anatomiques en cours de travail sur le patient 13 lui permettant de mieux comprendre l'environnement immédiat lors de son travail en bouche. Cet usinage peut se faire par soustraction (usinage conventionnel par fraisage) ou par addition (usinage non conventionnel comme la fusion laser ou la stéréo lithographie).

La figure 2 représente l'invention sous la forme d'un prototype dont une partie a déjà été réalisée. Dans le cas présenté, il est utilisé une caméra de lecture endobuccale 4 en stéréo scopie passive et à éclairage spécifique 5 pour mesurer la partie visible de la bouche (les dents et la gencive). L'unité centrale 3 est puissante et conventionnelle mais les logiciels sont spécifiques à l'invention. Les lunettes utilisées sont les « Google Glass » 1 classiques auxquelles sont fixés des accéléromètres et deux caméras. La machine-outil 17 est une machine par enlèvement de matière adaptée par le laboratoire de l'inventeur.

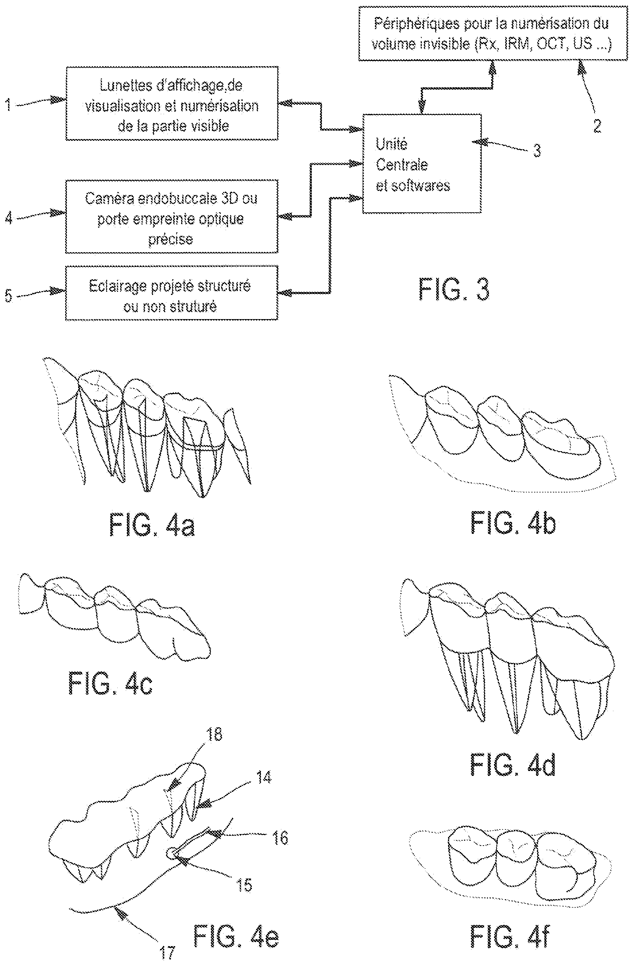

La figure 3 est importante car elle est la représentation en diagramme du coeur du dispositif, objet de l'invention.

Y sont représentées les lunettes de visualisation à réalité augmentée 1 permettant au clinicien de voir l'objet complémentarisé, c'est-à-dire son champ opératoire visible en vision directe et les parties visible et invisible parfaitement corrélées et numérisées sous la forme d'un seul objet virtuel confondu avec la vision directe. Les périphériques 2 en mesure de transmettre les informations sur la partie invisible de la bouche sont connectés ou non directement sur l'unité centrale et mettent à disposition ces information à priori (RX ...) ou en temps réel (ultrason ...). L'unité centrale 3 communique en permanence avec les lunettes afin que l'objet complémentarisé puisse être vu sous différent angles : les logiciels s'appuient sur les nuages de points 3D commun entre la vue de la partie invisible mémorisée et le vue 3D qu'observe le clinicien via les caméras que porte les lunettes à réalité augmentée.

L'objet complémentarisé doit donc être considéré comme un objet stable dans un repère orthonormé par rapport aux caméras des lunettes à réalité augmentée. Cet objet représente une sorte de volume plus ou moins cubique autour duquel tourne l'observateur. Ce sont les références communes ou d'autres indexes adjoints (cales de repérage) qui permettent au clinicien de tourner autour de l'objet complémentarisé virtuel comme il le ferait avec un hologramme.

Pour fiabiliser cette mise en correspondance des deux nuages de points, il apparait utile de faire un relevé plus précis de la surface visible que ne le fait les caméras portées par les lunettes à réalité augmentée. Pour cela est adjointe une caméra endobuccale 4 permettant la numérisation précise de la surface visible, la caméra utilisant ou n'utilisant pas de lumière structurée, l'éclairage, spécifique ou non, permettant une vision optimale de la bouche des dents et de la gencive.

De même pour apporter un complément non négligeable à la facette diagnostic de la présente invention, le dispositif de l'invention comprend un éclairage spécifique 5 optimisant la lecture des tissus dentaires durs (lumière blanche et bleue) mais aussi permettant de mettre en évidence certaines pathologies des tissus durs ou des tissus mous (fluorescence, phosphorescence, réaction au rayonnement IR, mixte IR/proche UV ...) .

Lors de la réalisation de ces actes cliniques, avantageusement et selon une caractéristique additionnelle du dispositif selon l'invention, des indications 3 sur l'état physiologique du patient peuvent apparaitre dans le champ opératoire. Il est en effet intéressant de connaître l'état cardiaque ou autres informations générales lors d'intervention particulièrement traumatisantes.

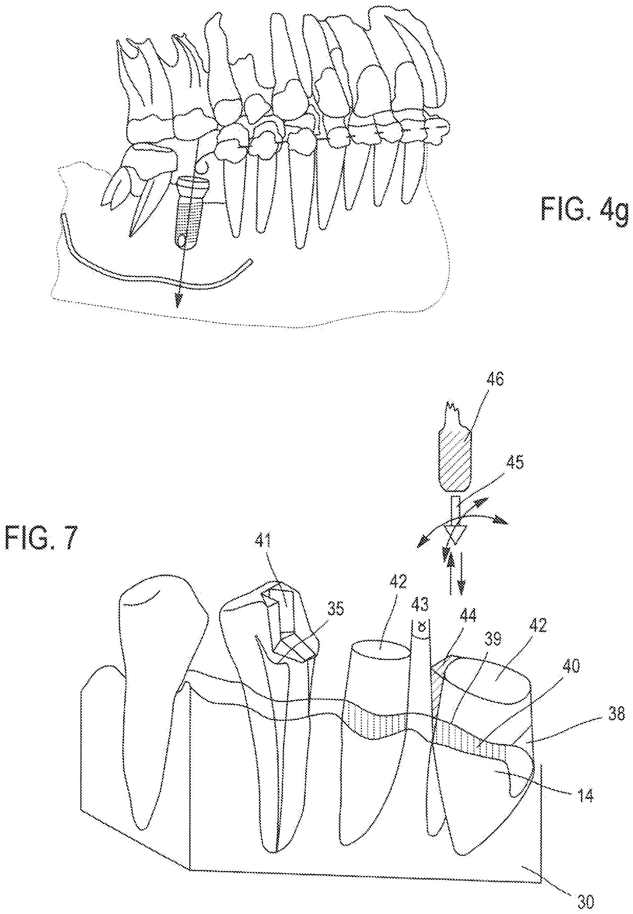

La figure 4 est une représentation imagée des étapes de construction de l'image 2-3D complémentarisé. Dans un premier temps le clinicien réalise une vue 2D, 2D 1/2 ou 3D (figure 4a) grâce au périphérique 2. Une vue 2D (par exemple RVG), une vue 2D 1/2 (tomo, cône beam, OCT ou scanner) ou mieux, une vue 3D (IRM, ultrason) permet de disposer des informations sur les structures invisibles. Comme les racines 14 ou les couronnes des dents 15, ces informations seront orientées vers les tissus durs en radiologie ou vers les tissus mous en IRM, le cône beam étant un bon compromis entre les deux. Le clinicien regarde son champ opératoire dans la bouche. Les lunettes à réalité augmentée porte des caméras stéréoscopique permettant de visualiser la partie visible de la bouche en 3D, c'est-à-dire les couronnes de dents 16 et la surface de la gencive 17 (figure 4b). Il peut aussi utiliser une caméra/scanner de lecture endobuccale 4 s'il veut avoir une grande précision dans sa lecture de la partie visible de l'image complémentarisée. C'est le cas de l'image qui est présentée en figure 4b. L'unité centrale va chercher les nuages communs 18 à la première image de la partie invisible (ici radiologique 4a) et à la deuxième image de la partie visible (ici à l'aide de notre scanner condor 4b). Il en ressort un nuage de point commun (figure 4c). Ce nuage correspond aux couronnes dentaires 19 car elles sont indéformables et présentes dans les deux parties visible 16 et invisible 15. A partir de ce nuage de point commun, le logiciel présent dans l'unité centrale va rapprocher les deux structures et les confondre au niveau du nuage de point pour former un seul objet volumique 3D ou objet complémentarisé 20 réunissant la partie visible 16 et invisible. C'est cet objet (figure 4d) qui s'affichera sur les lunettes à réalité augmentée. Le dentiste voit donc dans la bouche de son patient la partie visible et la partie invisible ce qui lui permet de traiter non seulement les couronnes mais aussi les racines de dents et les structures osseuses du maxillaire.

Il reste alors au logiciel de l'unité centrale 3 de suivre les mouvements du regard du dentiste pour lui permettre de contourner cet objet complémentarisé. Pour ce faire les caméras situées sur les lunettes vont continuer à suivre les différentes orientations que prend le nuage de point 18 par rapport au regard des caméras donc de celui du dentiste. Il s'en suivra recalage permanent de l'image virtuelle 3D complémentarisé affiché sur les lunettes du praticien 1 en information complémentaire de celle qu'il observe naturellement sur son site clinique. Ce recalage se fera en permanence et au fur et à mesure du déplacement de son regard.

Alors que la vue précédente était une vue linguale, la vue suivante (figure 4e) est une vue vestibulaire. Le clinicien a détourné son regard et voit les dents sur une autre face. Sur cette vue les racines vestibulaires sont courtes 21 car il a un regard plus plongeant. L'objet complémentarisé 20 composé de la partie visible et invisible respecte le mouvement du regard et permet de découvrir l'autre face de l'image 3D. Ceci est particulièrement intéressant car il est possible de voir l'émergence du trou sous mentonnier 22 et la sortie des nerfs et des vaisseaux sanguin 23.

Selon le même principe de la triple fusion caméra endobuccale / RX / vue en réalité augmentée, caractéristique additionnelle du dispositif selon l'invention, il est possible de connaître avec encore plus de précision l'environnement nerveux, veines, artères et structures anatomique.

Le dentiste sait donc exactement à quel endroit il doit piquer pour avoir une anesthésie parfaite du secteur antérieur (incisif et canin). Il peut voir aussi le rebord osseux de la mandibule 24 très important pour l'implantologie.

Il va de soi qu'une vue occlusale, sans effet de transparence pour le paquet vasculo-nerveux, respecte la surface visible qui reste dominante sur la surface invisible (figure 4f).

Cette invention permet de voir la totalité de l'anatomie dentaire directement dans la bouche, sur le site d'action clinique, sans avoir à déporter son regard ou faire des ajustements subjectifs pour savoir où se trouvent ces éléments anatomiques. L'acte devient précis et sécurisé.

La figure 4g montre la vue de l'objet complémentarisé associant la partie visible et invisible en un seul ensemble.

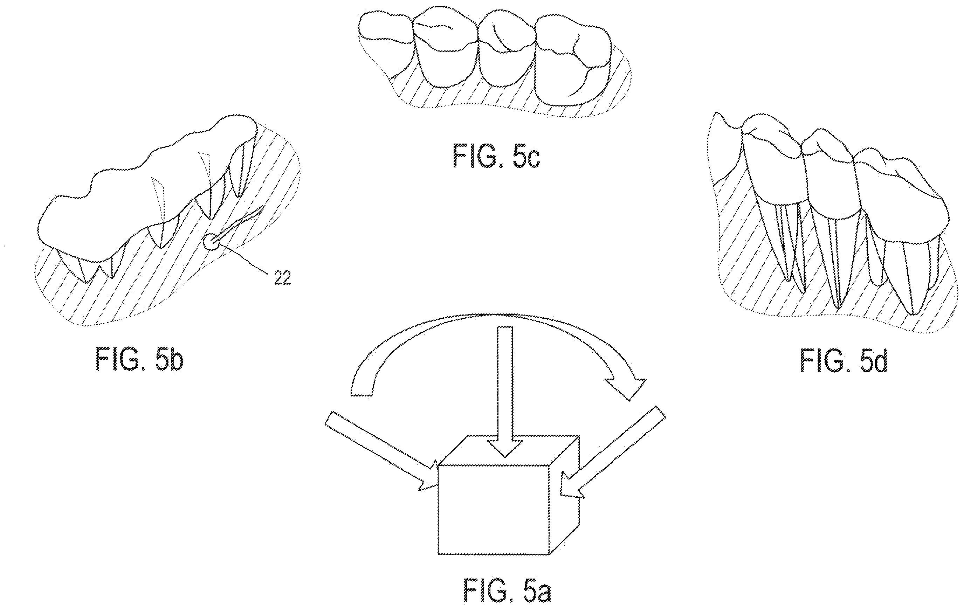

La figure 5 illustre l'effet du déplacement du regard du clinicien (5a) sur la vision observée au travers des lunettes à réalité augmentée en linguale (5b), occlusale (5c) ou vestibulaire (5d). Lorsqu'il déplace son regard, il est capable de voir l'intérieur de l'objet complémentarisé, invisible normalement, soit du côté vestibulaire, soit du côté lingual, soit du côté occlusal, ce qui lui permet de mieux appréhender la présence de composants anatomiques importants comme par exemple, à la figure 5d, l'émergence du nerf sous mentonnier 22.

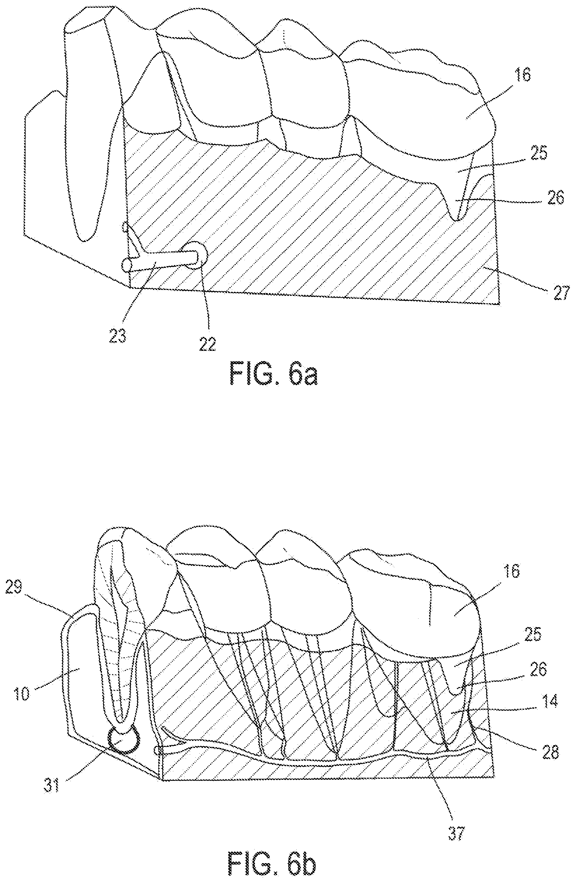

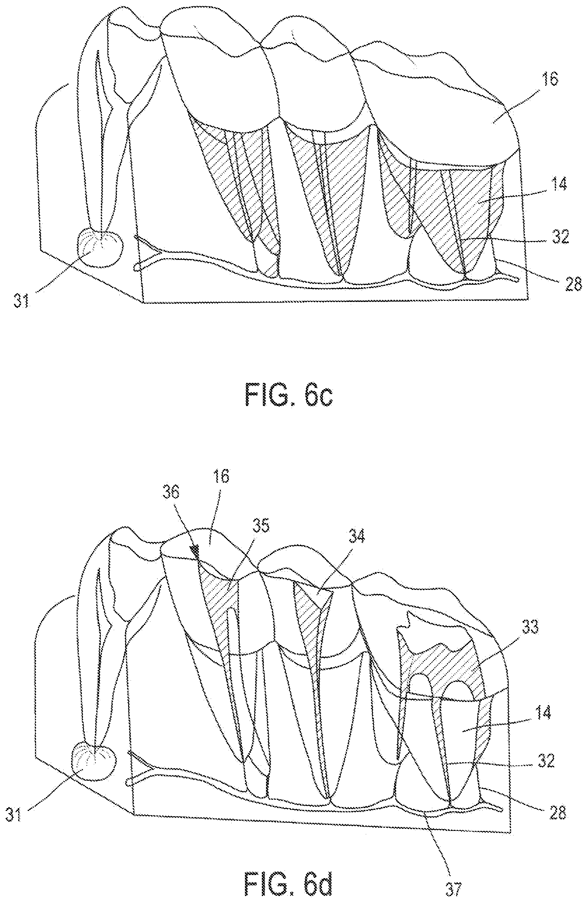

La figure 6 illustre l'effet de la variation du coefficient ou indice de transparence.

Sur la figure 6a, la gencive est supprimée sur un plan le plus proche de l'observateur mais l'os est visible. Sont visibles la couronne de la dent 16, le départ de la racine 25, le déchaussement de la racine 26 et la surface osseuse 27. Il est aussi possible de voir le trou mentonnier 22 si important pour les anesthésies et l'émergence du nerf 23.

Sur la figure 6b, qui est un plan plus profond, sont visibles la couronne 16, le départ de la racine 25 et son déchaussement 26. Sont visibles en plus et par transparence dans l'os, la racine 14 et le nerf qui innerve la dent 28. Comme le montre la section à gauche, l'os cortical 29 a été supprimé au profit de l'os médullaire 30 ce qui permet aussi de voir un kyste ou un granulome 31.

Sur la figure 6c, où l'os médullaire a été rendu transparent, le clinicien peut voir distinctement dans la bouche de son patient, dans le prolongement de chaque couronne 16 la racine des dents 14 mais aussi le nerf hors 28 et dans la dent le canal radiculaire 32 le renferment dans le paquet vasculo-nerveux. Le granulome ou le kyste sont également plus visible 31.

Enfin dans le dernier plan choisi dans cet exemple (qui n'est pas limitatif) sont distinctement visibles le canal coronaire 32 connecté ici au nerf et aux vaisseaux externes à la dent 28 mais aussi la pulpe coronaire de dents multiradiculées 33 et monoradiculées 34, 35, ce qui, évidemment permet de connaître parfaitement la position des cornes pulpaires 36.

En effet si l'objet 3D complémentarisé est unique, il conserve la connaissance de la partie visible et invisible. Le dentiste saura donc exactement l'endroit où il doit ouvrir la dent et pénétrer dans la racine 36 pour atteindre le nerf 37 avec un minimum de délabrement pour la dent. Il en serait de même dans la structure osseuse si le clinicien voulait atteindre un granulome 31, un kyste ou une tumeur.

Ces différents plans peuvent être choisis librement au pied, au clavier ou à la voie.

De même des indications plus locales peuvent lui être adressées. Ce peut être des indications sur l'état de son travail en cours ou après sa réalisation. Par exemple, à la figure 7 sont indiquées les contre-dépouilles 38 lors d'une préparation de prothèses dentaires ou la pose d'un implant en indiquant quelle action et à quel niveau faire une retouche ou une modification du travail pour garantir une bonne réalisation prothétique. Cette indication apparait sous la forme d'une surimpression de couleur ou de texture sur la zone à travailler. Elle disparait lorsque le travail effectué a répondu au besoin clinique.

De même sur cette figure est indiqué la forme de la préparation sous-gingivale invisible lorsqu'elle est recouverte par la gencive. La partie supra-gingivale est visible directement dans la bouche, la partie juxta-gingivale difficilement appréhendable par les méthodes directes alors que dans cette invention elle est très nettement visible 38, comme l'est la partie sous-gingivale 40. Cela permet au clinicien de savoir parfaitement s'il a des retouches à faire. Lorsqu'il prépare un inlay/onlay 41 des indications lui sont données directement en bouche en réalité augmentée sur sa préparation, indications qui disparaitront lorsque la préparation sera réalisée correctement.

Il en sera de même lors de réalisation de bridge. Le calcul de l'axe d'insertion 42 issu de l'analyse par exemple des barycentres, lui indiqueront le respect de l'angle à réaliser 43, de la zone à retoucher 44 mais aussi de l'angle que sa fraise 46 doit adopter si elle est équipée d'un détecteur de position spatiale en 3D 45.

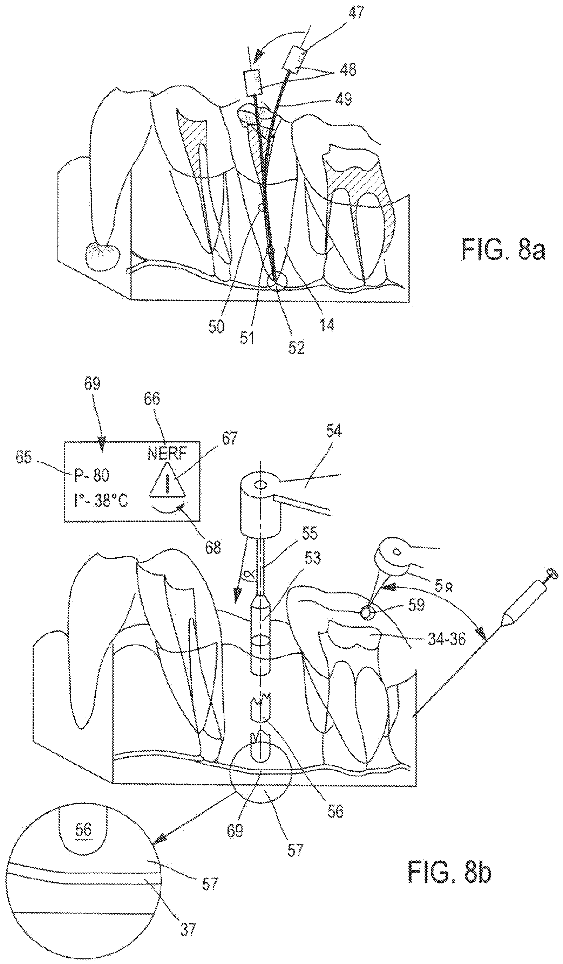

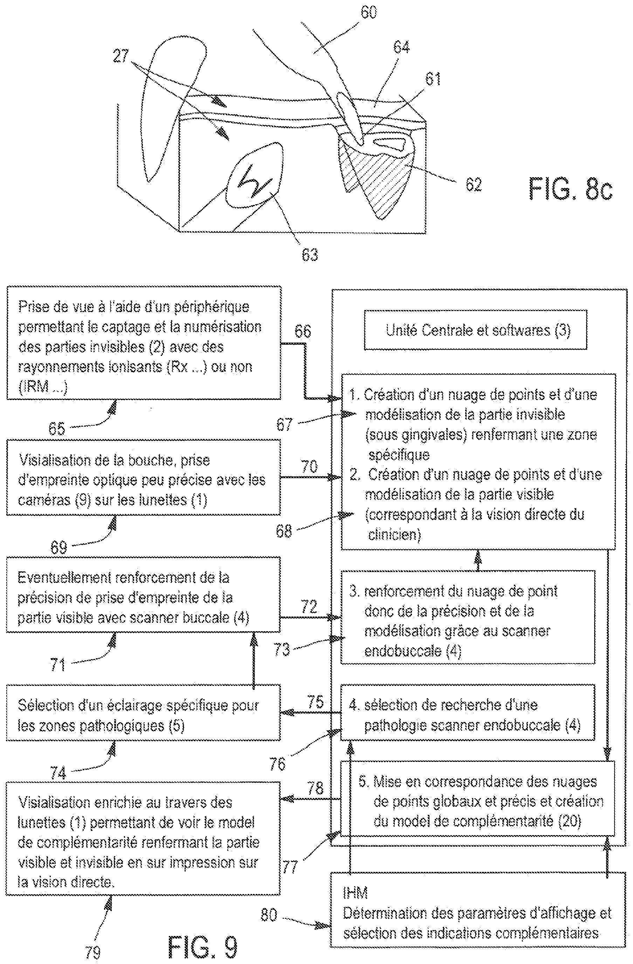

Comme illustré à la figure 8, la présente invention permet d'associer à l'objet complémentarisé le suivi dynamique des instruments qu'utilise le dentiste ou le chirurgien lorsqu'il procède à un acte de traitement radiculaire, à des actes chirurgicaux comme une extraction dentaire ou encore la pose d'implant.

Il est possible de suivre directement en bouche et en temps réel, sur le champ opératoire et dans la partie invisible de l'objet complémentarisé, sans avoir à détourner le regard, son acte opératoire et le mouvement des instruments qu'il utilise dans un même référentiel grâce aux lunettes à réalité augmentée.

Le clinicien peut suivre dans le temps les déplacements du nuage de points ou les modélisations caractéristiques ou mémorisés donc connu à priori de ses instruments de travail dans l'espace buccal.

Ainsi et avantageusement et selon une caractéristique additionnelle du dispositif selon l'invention, comme nous le voyons dans la figure 8a, ces instruments sont manipulés de la manière suivante :

- La première étape consiste à repérer dans l'espace l'instrument utilisé, au départ de l'opération, en utilisant les caméras 9 situées sur les lunettes à réalité augmentée 1 et grâce à des références spécifiques 47 (par exemple une tête d'instrument d'une forme particulière ou un code bar).
- L'instrument utilisé est recherché dans une bibliothèque renfermant un ensemble de forme d'instruments mémorisés. Dans ce cas, les instruments sont modélisés par un logiciel en se basant sur son image avec une identification particulière les rendant facilement identifiables. Ce peut être un repère fixé sur la manche de l'instrument ou un message wifi ou magnétique, sans que cela soit limitatif, le principe de l'invention étant une reconnaissance de l'objet utilisé par le clinicien.
- Il est aussi possible de l'identifier et d'indiquer manuellement sa position sur un écran. Cela à l'avantage de faciliter le travail du traitement d'image, mais oblige le praticien à intervenir sur l'écran annexe.
- La deuxième étape consiste à suivre le mouvement de cet instrument connu et positionné dans l'espace de l'objet complémentarisé. Ce suivi est possible, en temps réel ou quasi réel, par les caméras de visualisation 9 qui repèrent les mouvements des points des repères identifiés précédemment dans l'espace par le logiciel de traitement d'image.
- Ce suivi est donc une mise en correspondance dynamique en temps réel ou légèrement déféré de l'instrument et de l'objet complémentarisé par le suivi de ces repères, caractéristiques de l'instrument utilisé, et les zones indéformables caractéristiques de l'objet complémentarisé.
- Il peut être adjoint une indication sonore ou visuelle s'il y a risque d'atteindre des zones sensibles (veines ou nerfs ...) .
- Il peut aussi être adjoint une indication visuelle ou sonore pour que le geste du clinicien soit précis et dans la bonne direction (dents incluses ou granulomes, voire cancer) avec des informations permettant une orientation idéale, voire automatique, ou l'apparition d'un zoom pour mieux visualiser s'il existe un risque.

Le praticien a donc une vue du déplacement de ces instruments dans l'objet complémentarisé comme s'il utilisait une radio dynamique. Ceci est particulièrement intéressant car il peut suivre la progression sans aucune radiation ionisante.

Comme le montre la figure 8a, l'instrument utilisé se compose, par exemple mais ceci n'est pas limitatif, de deux parties, l'une indéformable 48 contenant les éléments de repérage spatiaux 47 permettant de reconnaître et de suivre l'objet dans ses déplacements dans l'espace et une autre partie, correspondant à la zone active 49, efficace sur le plan clinique. Ces zones peuvent être confondues.

Ainsi et avantageusement et selon une caractéristique additionnelle du dispositif selon l'invention, il aura deux possibilités.

Soit l'instrument est déformable comme, par exemple, une broche 48, une sonde ou une mèche des traitements endodontiques. Dans ce cas l'instrument est corrélé à la densité ou au contraste, et ceci n'est donné qu'à titre d'exemple, de la zone dans laquelle il est introduit dans l'objet complémentaire. Cette zone de même qualité optique 50, dans l'image 3D (zone de progression) peut être automatiquement repérée ou indiquée par l'opérateur. L'instrument se déformera de telles sortes qu'il suive cette densité ou ce contraste 51. Par exemple un instrument canalaire déformable sera introduit dans une chambre 50 puis un canal dentaire 51 qui ont une densité, un niveau de gris très particulier par rapport à la dentine de la racine 14. Cet instrument, que le logiciel a reconnu et modélisé, se déformera pour suivre la densité ou le contraste caractéristique du canal.

Soit l'instrument utilisé est indéformable comme, par exemple dans la figure 8b, une fraise 59 ou une aiguille 58. Il traverse l'objet complémentarisé sans tenir compte des densités ou contrastes caractérisant différentes régions anatomiques. Le logiciel est à même d'anticiper ce mouvement instrumental et les risques de danger qui l'accompagne (rencontre d'un nerf ou d'un vaisseau, voir le risque de perforer un sinus dans le maxillaire supérieur).

Ainsi et avantageusement et selon une caractéristique additionnelle du dispositif selon l'invention, les instruments indéformables ou déformables sont mémorisés dans une bibliothèque spécifique. Ceci permet au clinicien de les sélectionner manuellement ou de lancer une recherche automatique. Les caractéristiques géométriques de l'instrument ayant été mémorisées, son intégration à l'image renferment l'objet de complémentarité est particulièrement aisée.

Cette reconnaissance peut aussi se faire automatiquement grâce à la lecture de référence sous formes diverses (code bars ...) Cette identification étant faite cela conduit à une connaissance automatique des données géométriques de l'instrument, son identification plus facile dans l'image visualisée par les caméras 9 des lunettes de visualisation et le suivi de ses mouvements dans l'objet complémentarisé.

Ainsi le suivi des mouvements de l'instrument déformable ou indéformable se fera par des moyens optiques, mais aussi par toute technique de repérage spatial (accéléromètre, gyroscope, magnétomètre, ultrason, IR, GPS ...).

Comme le montre la figure 8b, en implantologie il est possible d'indiquer la meilleure position et le meilleur axe d'insertion du foret préparant le site de l'implant. Si l'outil 54 est muni d'un repérage tridimensionnel, par exemple tel que celui du brevet FR n° 92.08128, le logiciel indique en réalité augmentée sur les lunettes d'affichage, directement à la hauteur du foret ou de la pièce à main (au choix), l'axe à respecter 55, et émet un signal sonore à tonicité variable en fonction de l'exactitude ou de la dérive 68 de la position ou de la proximité d'un élément anatomique 66. L'information locale peut aussi apparaitre en surimposition sur la paire de lunettes à réalité augmentée 69 associée aux logiciels présents dans l'unité centrale 4. Elle indique toutes les informations 69 en temps réel et guide pour axer parfaitement le forage 65-68 et l'arrêter lorsqu'il est assez profond 67. De même, et toujours en implantologie, l'invention indique quel type d'implant, quelle forme ou quelle marque répond le mieux à l'environnement tridimensionnel analysé grâce à la triple fusion 9 image précise / image en réalité augmentée / image RX.

Dans certains cas aucun implant ou aucune prothèse ne correspond à l'environnement idéal visualisé en réalité augmentée par l'opérateur et il est nécessaire de faire l'implant ou la prothèse sur mesure. L'unité centrale 3 peut alors être connectée à une machine outils à commande numérique 12 pour réaliser cet implant ou cette prothèse spécifique, unitaire ou pleurale.

Lorsque le foret d'implant approche une zone dangereuse (ici un nerf 37), il est possible d'avoir un agrandissement (automatique ou sur demande) de la zone à risque 57. Ceci permet de mieux contrôler le mouvement du foret 56 par rapport au nerf 37.

Enfin, comme visible à la figure 8c, il est possible de suivre un acte chirurgical dans l'objet complémentarisé. Par exemple, l'objet indéformable 60 utilisé est un élévateur permettant d'arriver très exactement au niveau de la racine 62 qui normalement est invisible dans la bouche. Grâce à l'invention, il est possible de la voir et de suivre dans l'objet complémentarisé la progression de la tête 61 de l'élévateur. Il en est de même pour la recherche d'une dent incluse 63 située sous l'os et la gencive 27.

Il va de soi que cette application de l'invention ne se limite pas à la dentisterie mais peut s'appliquer à toute opération chirurgicale sur le corps ou en médecine vétérinaire.

La figure 9 explique, grâce à un diagramme, les différentes étapes cliniques de manipulation.

La première étape consiste à récupérer, du périphérique 2, les informations de la partie invisible, c'est-à-dire une vue 2D 1/2 ou 3D des tissus sous-jacents 65. Cette vue correspond à un nuage de points (les voxels) représentant les dents (couronnes 15, racines 14 et conduits des tissus pulpaires), l'os médullaire et cortical 24, les vaisseaux et les nerfs 23 mais aussi la géographie anatomique de ses composants invisibles. Un fichier renfermant ces voxels sous la forme d'un nuage de points 66 est adressé à l'unité centrale de traitement 3 dans un fichier.

Le fichier de la partie invisible 67 reçu par l'unité centrale, le praticien peut prendre ses lunettes 1 et visualiser sa zone de travail dans la bouche de son patient 8 et, à l'aide de l'IHM, mettre en fonction les lunettes à réalité augmentée. Ceci lui permet de récupérer un deuxième nuage de points 68 de la partie visible dans la bouche de son patient grâce à l'action des caméras externes ou/et d'empreintes optiques 69 et leurs connexions 70 avec l'unité centrale 3.

S'il le souhaite l'utilisateur peut renforcer la précision de son nuage de points en utilisant des caméras d'empreintes optiques 4 avec ou sans lumière structurée 71. Cette action permet d'envoyer un nuage de points précis à l'unité centrale 72, ce qui renforcera 73 la qualité du nuage des caméras sur les lunettes à réalité augmentée 68 en s'appuyant sur les zones communes au nuage de points de l'objet complémentarisé et de ses parties visibles 68 et invisibles 67.

En utilisant un éclairage spécifique 74 de la caméra endobuccale, il peut enrichir les informations 75 reçues par l'unité centrale, principalement dans le domaine du diagnostic 76.

A ce stade, il disposera de deux nuages de points 67, 68 renforcés par des informations précises 73 et éventuellement des informations diagnostics 76. Il y a alors fusion au niveau de l'unité centrale 77 et création de l'objet complémentarisé.

Cet objet est alors transmis 78 aux lunettes à réalité augmentée afin que l'objet complémentarisé s'affiche dans le champ de vision 78 et que le praticien puisse voir dans la bouche de son patient les parties visibles et invisibles de son champ opératoire 79.

L'ensemble de ces commandes sont sous le contrôle d'un IRM spécifique manuel ou vocal 80.

La connexion à l'IHM et le geste du praticien est d'autant plus libre et sa vision plus directe que la connexion entre ces différentes unités se fera par un long câble ou sans câble (Wifi, Bluetooth..). Les connexions sans fil sont préférées, mais ceci n'est pas limitatif de l'invention. Si les connexions se font par câbles, il est préférable d'utiliser, par exemple, une connexion USB autoalimentée.

Si la connexion est sans fil, elle peut être par exemple en mode Wifi, mais ceci n'est pas limitatif selon l'invention. Dans ce cas, si elle n'est pas présente originellement dans le périphérique, l'antenne sera ajoutée dans la caméra 4, les lunettes à réalité augmentée 1 et les autres périphériques 2. De même, sur l'ordinateur 3 ou éventuellement sur un boîtier intermédiaire sera introduite dans la connexion USB une antenne d'envoi et de réception des données correspondant aux ordres donnés par le dentiste 6 à l'aide de son micro 11, par le programme situé dans l'ordinateur 3 ou le boîtier intermédiaire s'il ne dispose pas de cette fonction de transmission. Cette disposition permettra une communication rapide, conviviale et aisée quelques soient les configurations des cabinets dentaires.

Comme visible sur le diagramme de la figure 9, il est possible d'envoyer ou de recevoir d'autres informations importantes pouvant permettre au clinicien 6 de travailler dans le confort et la précision. Ceci est rendu possible grâce à la création de l'objet complémentarisé et de la vue au travers des lunettes à réalité augmentée 1, sans qu'il quitte des yeux son champ opératoire 8.

Ainsi et avantageusement et selon une caractéristique additionnelle du dispositif selon l'invention, le praticien recevra des informations non seulement statiques par fusion et la création d'un objet complémentarisé 20 mais aussi dynamiques en suivant les mouvements dans le temps des appareils intervenant sur cet objet complémentarisé. A chaque instant un recalage se fera entre l'objet complémentarisé et les déplacements ou les variations imprimées par les actions du clinicien 6 et visualisées en réalité augmentée au travers de ses lunettes 1.

Tout ce montage est réalisable en suivant une procédure mathématique précise qui peut être une présentation des éléments logiciels utilisable pour la fusion des deux parties visibles et invisibles pour la création de l'objet complémentarisé.

Il ne s'agit que d'un exemple de calcul de trace optique par mise en correspondance des points d'intérêt. Il permet d'expliquer comment construire un objet complémentarisé à partir des images reçu du périphérique 2 et celles 2D issus de la lecture à l'aide des caméras 9 sur les lunettes à réalité augmentée 1 et/ou celles issue des caméras de prise d'empreinte optique 4.

La recherche des traces optiques de points 3D remarquables entre la partie commune du nuage visible et invisible se fait par recherche de points d'intérêt sur toutes les images acquises, puis par recherche des correspondances entre les points d'intérêt de différentes images.

Plusieurs schémas sont possibles :

Un premier schéma est l'alignement optique d'angles. L'idée générale est de calculer des points remarquables (angles) sur une image, puis de suivre ces points sur les images suivantes sans avoir à les redétecter. La phase de suivi se poursuit tant qu'un certain pourcentage de points remarquables de la première image est encore détectable (typiquement 70%) ; en-deçà de ce seuil, une nouvelle phase de détection de points remarquables est menée sur l'image suivante.

La détection d'angles se fait en calculant pour tout pixel (x,y) la matrice 2*2

$$C = \begin{bmatrix} \sum_W \left(\frac{\partial I}{\partial x}\right)^2 & \sum_W \left(\frac{\partial I}{\partial x}\right) \cdot \left(\frac{\partial I}{\partial y}\right) \\ \sum_W \left(\frac{\partial I}{\partial x}\right) \cdot \left(\frac{\partial I}{\partial y}\right) & \sum_W \left(\frac{\partial I}{\partial y}\right)^2 \end{bmatrix}$$

, où I désigne l'intensité en (x,y) de l'image et W un voisinage de (x,y). Soit $\lambda_1$ et $\lambda_2$ les 2 valeurs propres de cette matrice ; si ces 2 valeurs sont supérieures à un certain seuil (typiquement 0,15), le point est considéré comme un point remarquable.

Pour l'alignement, est recherché, entre 2 images i et i+1 et pour chaque point remarquable, le déplacement $d=(d_x,d_y)$ qui minimise $\sum_W (I_i(x,y) - I_{i+1}(x + d_x, y + d_y))^2$. Ce déplacement est calculé par $d=C^{-1}.b$, avec C la matrice 2*2 précédemment évoquée et $b = \sum_W \begin{bmatrix} (I_i(x,y) - I_{i+1}(x,y)).I_i(x,y) \\ (I_i(x,y) - I_{i+1}(x,y)).I_{i+1}(x,y) \end{bmatrix}$ . Cette technique d'alignement optique étant fiable sur de petits déplacements, les éventuels grands déplacements sont déterminés en calculant séquentiellement le déplacement d sur une pyramide d'images (d'une version très subsamplée des images jusqu'à la résolution initiale).

Les techniques ci-dessus se basent sur l'hypothèse implicite que le flux d'images est cohérent, i.e. le déplacement entre 2 images successives est faible, et 2 images successives sont de qualité suffisante pour que l'on retrouve une quantité satisfaisante de points en correspondance (au moins 30).

En ce qui concerne le déplacement entre 2 images, l'acquisition des images est à une fréquence classique de flux vidéo. Il concerne donc un déplacement très faible entre 2 images. Pour un déplacement plus conséquent qui entraînerait une impossibilité de trouver des points correspondants avec les images précédentes, il sera possible de générer une nouvelle région.

Pour ce qui est de la qualité insuffisante d'une image (dans l'éventualité d'une image floue par exemple), la phase de mise en correspondance joue le rôle de filtre puisqu'il est clair que très peu de points correspondants seront retrouvés. L'image sera alors stockée sans être traitée, et on attendra l'image suivante qui aura un nombre suffisant de points correspondants.

Un second schéma concerne les points invariants et la mise en correspondance aux moindres carrés.

Les points d'intérêt sont recherchés sur les images par des techniques bien connues, qui recherchent des points restant invariant par changement d'échelle et d'illumination. Ces techniques présentent l'avantage de pouvoir calculer des descripteurs morphologiques pour chaque point d'intérêt.

La mise en correspondance entre points d'intérêt pour une paire d'images donnée est effectuée en recherchant, pour tout point d'intérêt $x_{i1}$ de l'image 1, le point d'intérêt $x_{i2}$ de l'image 2 minimisant la distance à $x_{i1}$ aux moindres

carrés en termes de descripteurs. Pour éviter les fausses correspondances ou les outliers, on calculera au préalable la matrice fondamentale F entre les images 1 et 2 (qui lie les couples de points d'intérêt par la relation $x_{i1} \cdot F \cdot x_{i2}^t = 0$) . Si, pour un couple de points d'intérêt $x_{i1}$ et $x_{i2}$ potentiellement correspondants aux moindres carrés, le produit $x_{i1} \cdot F \cdot x_{i2}^t$ est supérieur à $10^{-5}$, ce couple est alors rejeté.

La recherche de trace optique se fait alors par transition lors de l'acquisition d'une nouvelle image. Lorsque l'image $I_j$ est acquise, cela suppose que le calcul de trace optique a été effectué pour toutes les images précédentes $I_1 \dots I_{j-1}$. Sont calculé alors les points d'intérêt de $I_j$, que l'on met en correspondance avec l'image $I_{j-1}$. Les traces optiques sont alors complétées par transition en remarquant que si $x_{ij}$ est en correspondance avec $x_{ij-1}$ quit est en correspondance avec $x_{ij-2}$, alors $x_{ij}$ est en correspondance avec $x_{ij-2}$.

Un troisième schéma concerne les forts gradients et la mise en correspondance par corrélation

Sont considérés comme points d'intérêt d'une image l'ensemble des points où les variations d'intensité sont importantes. En pratique, pour chaque point de l'image considérée est calculé l'écart-type des intensités dans un voisinage de 20*20 pixels autour de ce point. Si l'écart-type est supérieur à un certain seuil (typiquement de l'ordre de 10, pour des intensités codées sur 8 bits), le point est alors considéré comme point d'intérêt.

La recherche de correspondances entre 2 images au niveau de leurs points d'intérêt se fait par une technique de corrélation par exemple de type Médicis (EP1756771 et EP0600128) .

Un algorithme de reconstruction 3D temps réel permet de suivre en dynamique le mouvement d'un instrument se déplaçant dans l'objet complémentarisé.

La modélisation 3D suit trois étapes. Dans la première étape, le nuage de points 3D obtenu par traitement des traces optiques est densifié grâce au calcul d'une fonction implicite f d'interpolation. Grâce à cette fonction implicite, la surface 3D interpolant les points est polygonalisée par la méthode par exemple de type Bloomenthal. Enfin, chaque polygone est texturé de manière très simple : par projection des points 3D délimitant le polygone sur les images ayant généré ces points, est délimitée une zone polygonale sur ces images. La texture sur ces zones polygonales est moyennée et attribuée au polygone.

La principale difficulté réside dans l'algorithme utilisé pour l'interpolation et le calcul de la fonction implicite. Cet algorithme est adapté de manière optimale à notre utilisation, car il permet une interpolation en temps réel et, contrairement aux autres techniques d'interpolation, il permet une interpolation dense à partir d'un nuage initial très épars, ce qui est très souvent le cas quand le travail concerne des objets avec peu de texture comme les dents.

L'interpolation générique à la base de cet algorithme, puis son utilisation en pratique dans un schéma multi-échelles, se réalise ainsi : Pi étant les points du nuage 3D (après estimation de la normale $\vec{n}$ en ces points), la fonction implicite $f: \mathbb{R}^3 \mapsto \mathbb{R}$ , basée sur des RadialBasis Functions (RBF) telle que les points X appartenant à la surface soient ceux pour lesquels f(X)=0, est choisie telle que :

$$f(x) = \sum_{p_i \in \mathcal{P}} [g_i(x) + \lambda_i] \cdot \Phi_\sigma(\|x - p_i\|) \quad ,$$

avec

$$\Phi_\sigma(x) = \Phi\left(\frac{x}{\sigma}\right), \phi(x) = (1 - r)^4 + (4r + 1)$$

Les inconnues à déterminer pour expliciter f sont donc les $g_i$ et les $\lambda_i$.

Connaissant le point Pi et sa normale $\vec{n_i}$, un système (u,v,w) est choisi tel que u et v soient perpendiculaires à la normale et w pointe dans la direction de la normale. Soit h une fonction de la forme $h(u, v) = Au^2 + Buv + Cv^2$, en pi, les coefficients A, B et C sont cherchés de manière à minimiser la quantité suivante $\sum_{p_j \in \mathcal{P}} \Phi_\sigma(\|p_j - p_i\|) \cdot (w_j - h(u_j, v_j))^2$ et gi(x) est calculé par $g_i(x) = w - h(u, v)$.

Sachant que $f(P_i) = 0$, $\forall P_i$, $\lambda_i$ est estimé par une simple résolution de système linéaire.

Interpolation multi-échelles : L'interpolation générique est en fait menée sur des sous-ensembles de points pour améliorer grandement la précision de l'interpolation. Après avoir construit un ensemble $\{\mathcal{P}_0, \dots, \mathcal{P}_k\}$ en prenant l'ensemble $\mathcal{P}_0$ comme un parallélépipède englobant l'ensemble des points Pi, une subdivision de parallélépipèdes en 8 petits parallélépipèdes est effectuée entre 2 niveaux successifs k-1 et k.

La fonction f est calculée par une procédure itérative. On part de $f^0=-1$, puis on itère sur les ensembles $\mathcal{P}_k$ en mettant à jour f :

$$f^k(x) = f^{k-1}(x) + o^k(x), o^k(x) = \sum_{p_i^k \epsilon \mathcal{P}_k} \left( g_i^k(x) + \lambda_i^k \right) \cdot \Phi_{\sigma^k}\left(\|x - p_i^k\|\right)$$

Les $g_i^k$ sont déterminés comme décrit précédemment sur l'ensemble $\mathcal{P}_k$, et les $\lambda_i$ sont calculés en résolvant

$$f^{k-1}\left(p_i^k\right) + \ o^k\left(p_i^k\right) = 0 \ .$$

Les $\sigma^k$ sont mis à jour de telle sorte que $\sigma^{k+1} = \sigma^k/2$, et le nombre de niveaux à construire est défini par M = $-log_2(\sigma^0/2\sigma^1)$.

La manipulation d'un tel système est extrêmement simple car son paramétrage est réputé fixe et non modifiable par l'opérateur à l'exception de certaines sélections pré-manipulations ou d'autres (préétablies) nécessitant des précisions en cours de travail. Dans les premières on trouve par exemple la fiche du patient (fiche clinique) alors que dans l'autre on trouve les instruments qu'il peut utiliser (déformables ou non), la sélection du type de vision (par exemple avec ou sans l'os) ou encore le type de diagnostic visé (par exemple la carie ou les tumeurs qui n'auront pas le même type d'éclairage).

Cette fonction peut être pilotée par une succession d'actions automatiques conduisant au diagnostic désiré. Pour ce faire, l'opérateur (dentiste, prothésiste ou médecin) dispose d'un ordinateur lui indiquant les opérations que peut exécuter les lunettes à réalité augmentée et/ou les caméras (augmentée ou non d'une lecture précise avec le scanner endobuccal) en lui demandant de faire le choix entre une fonction et une autre.

Il est à noter que l'on privilégie les actions cliniques sur le type de matériel. Ainsi nous n'indiquons pas sur le menu déroulant ou en détection vocale lumière fluorescente mais carie détection.

Tout ou partie du traitement peut se faire au niveau des cartes incluses dans un système générique (portable ou ordinateur de bureau standard) ou dans un système spécifique comportant des cartes spécialement dédiées à l'application de traitement, de transmission et de visualisation des données. Cet ensemble peut être intégré à l'Unit ou séparé (par exemple dans un chariot).

La première étape consiste à collecter les images invisibles 2D, 2D 1/2 ou 3D issues du périphérique 2 et de les stocker en temps différé (RX, IR, IRM ...) ou en temps quasi réel (ultrason, OCT ...). Une fois le stockage effectué, le praticien lance la construction de l'objet complémentarisé 3D ou pseudo 3D.

La deuxième étape consiste à prendre ses lunettes à réalité augmentée 1 et à lancer la lecture des caméras 9. L'image réelle vue au travers des lunettes s'enrichie successivement :
Tout d'abord de la modélisation 3D de la partie visible construite avec les deux caméras 9 fixées sur les lunettes 1. Quoique cela soit possible, pour des questions de sécurité, il n'y a jamais élimination de la vision directe au profit de la représentation modélisée dans le champ de vision mais fusion entre la vue directe et cette modélisation issue du nuage de points.

Ensuite le clinicien appelle la vision de la partie invisible issue du périphérique choisi 2 qui, en s'appuyant sur les points communs, viendra compléter la vue modélisée des deux caméras 9 pour créer l'objet complémentarisé renfermant la partie visible et la partie invisible.

Si le clinicien souhaite avoir une vue parfaite en bonne définition de la vue visible, il a la possibilité d'effectuer un scannage complémentaire à l'aide d'une caméra de lecture optique endobuccale 4.

Eventuellement et pour faciliter la fusion des deux nuages de points, ceux issus de la lecture effectuée sur la partie invisible 2 et ceux issus de la lecture du visible des lunettes 1 et 9 et du scanner endobuccal 4, il peut indiquer à l'aide d'un instrument spécifique la zone commune aux deux fichiers (par exemple les couronnes des dents) directement sur le site ou sur un écran à proximité.

Il peut avoir aussi recours à une cale de calibration permettant d'homogénéiser les deux vues en terme dimensionnel, grâce au logiciel d'interpolation multi échelle. En effet dans certains cas, particulièrement lorsqu'il s'agit de corréler la (les) vue(s) 2D sur le nuage de points 3D de la vue visible, la correspondance est plus difficile. S'appuyant sur le référentiel joint dans la vue 2D invisible, cette cale permet plus facilement le travail de ce logiciel. Avantageusement, et selon l'invention, les LED peuvent jouer aussi un rôle important dans la corrélation des vues successives. En effet nous savons qu'il existe des méthodes basant les corrélations des vues sur des repères posés dans l'environnement mesuré ou utilisant la similitude retrouvée dans le nuage lui-même, voire même travaillant sur les flous de rebord de vues. Tous ces systèmes sont complexes car ils obligent soit à poser des repères sphériques dans la zone, opération complexe sur le plan clinique, ou à repérer des zones souvent sans relief ou avec un état de surface trop régulier. Le fait de balayer avec des LED de longueurs d'onde connues avec une imagerie 3D en couleur permet de simplifier et d'automatiser cette procédure. En effet un simple tracé coloré ou le collage d'une marque peut être repéré et visualisé automatiquement si nous avons pris soin d'utiliser un marquage ou un repérage utilisant une couleur complémentaire, identique additive ou soustractive de la longueur d'onde d'une (ou plusieurs) des LED de balayage. Le repérage sera donc fait par une simple mise en valeur chromatique du repère quel qu'il soit. Ce repérage, qui est toujours dans une même position sur l'objet quel que soit l'angle ou le zoom de nos empreintes optiques servira de référence de corrélation.

La fonction fusion pour objet complémentarisé peut être lancée à l'aide d'un bouton situé sur ses lunettes ou à proximité du siège clinique, d'une information verbale avec le micro 11 ou d'une pédale en communication avec l'ordinateur, et il peut l'arrêter lorsqu'il jugera qu'elle est correcte ou qu'il a fini son acte clinique. Pour cela il cesse la pression ou appuie une deuxième fois.

Les caméras 9 sur les lunettes 4 interpoleront en permanence le fichier invisible sur le fichier visible offrant une vue complète de la partie sus et sous gingivale au regard du clinicien directement en bouche et en temps réel ou quasi réel.

Il est à noter que cette opération de fusion visible/invisible peut se faire sur une modèle en plâtre dans un laboratoire, le prothésiste pouvant disposer de lunettes à réalité augmentée. Cela permet au prothésiste de disposer d'informations intéressantes quand il a à préparer des prothèses sous gingivales, des appareils amovibles nécessitant une connaissance des épaisseurs des tissus gingivaux et du relief osseux, des guides chirurgicaux et des implants préfabriqués ou sur mesure en implantologie grâce à la visualisation à la place du plâtre des organes sous-jacent (artères, ligne de finition sous-gingivale ...). Sans cette invention, l'opération serait impossible.

Le traitement logiciel permet de calculer en temps quasi réel, les coordonnées 3D (x, y, z) et la couleur de chacun des points mesurés en x, y et z. Nous obtenons un fichier 3D d'une arcade partielle ou complète en couleur associé à des informations de la partie invisible.

Les prises d'images successives des caméras situées sur les lunettes 1, véritable film de la zone à visualiser, permettent un relevé complet des informations nécessaires aux traitements numériques de l'ensemble ou d'une partie de l'objet à voir mais aussi à mesurer en vestibulaire, en lingual et en proximal s'il désire utiliser la fonction mesure stéréoscopique que peut offrir une caméra créant des nuages de points (FR 14.54774). Ces zones sont fusionnées automatiquement par le logiciel sur les vues précédentes imprécises en s'appuyant sur le même référentiel commun (par exemple les couronnes des dents. Cette même détection des zones communes peut se faire au niveau des courbes de modélisation (Nurbs, radial basis functions, ondelettes ....).

Si le praticien décide d'utiliser la fonction diagnostic il sélectionne sur l'ordinateur ou de manière verbale le type de diagnostic désiré, par exemple mélanome ou carie détection, la caméra lancera un balayage de longueurs d'ondes correspondant à la mise en évidence des zones intéressant les longueurs d'ondes présélectionnées et présentes sur une image 3D. En plus de cela, et grâce à l'analyse 3D de l'objet, le recouvrement des mesures dans le temps permettra de mieux suivre l'évolution de la dite pathologie. Il est en effet admis par les professionnels que l'étude d'une image suspecte peut être faite en 2D mais c'est surtout l'évolution de son volume et de sa couleur qui sert de référence au suivi dans le temps de sa dangerosité. Le fait de disposer d'un volume référé à un centre mathématique (comme par exemple le barycentre) permet de superposer les images sur un centre dépendant de l'objet et non pas de l'observateur afin d'en apprécier objectivement l'évolution du volume, l'analyse de la couleur venant se reporter sur une forme 3D, ce qui n'est pas le cas aujourd'hui des méthodes pratiquées sur des surfaces 2D ou celles utilisant des lumières ou les ondes structurées (OCT, scanner ou IRM).

De même, en sélectionnant certaines longueurs d'ondes émises par les LED présentes autour de la fenêtre de lecture et en augmentant leurs fréquences ou/et leurs intensités, nous pouvons reporter sur une image 3D la visualisation de certaines anatomies ou pathologies se situant à toute profondeur en plus de celles visualisées par le périphérique visualisant la partie invisible. La connaissance du volume nous donne une indication du positionnement de cette limite pathologique nous permettant d'en prévoir et d'en visualiser l'évolution. Il en est ainsi des réactions de fluorescence de certains tissus aux rayonnements bleus ou UV. La fluorescence apparait non seulement sur la surface mais dans la profondeur de la pathologie nous permettant d'apporter une aide à la thérapeutique à appliquer (exérèse des tissus pathologiques). Connaissant la pénétration de tel ou tel rayonnement, il nous est possible d'en apprécier l'importance et la profondeur par rapport à la surface réelle 3D analysée.

Il ressort de la description qui précède que la présente invention, répond parfaitement aux problèmes posés, en ce sens, qu'elle apporte une réelle réponse à la visualisation des zones visibles et invisibles et plus particulièrement à leurs réunions dans un même référentiel permettant la visualisation de l'objet complémentarisé directement en bouche sur le site clinique. Elle permet une analyse anatomique immédiate et pathologique des pathologies de la gencive et des tissus sous-jacents. Il ressort aussi de cette description, qu'elle permet, de résoudre les problèmes fondamentaux, comme le contrôle de l'acte clinique, ce d'autant qu'aucune méthode alternative n'a été proposée. Il va de soi que l'invention ne se limite pas au seul mode de mise en oeuvre de ce procédé, ni aux seules formes d'exécution de dispositif pour la mise en oeuvre de ce procédé, écrites ci-dessus à titre d'exemple. Elle embrasse, au contraire, toutes les variantes de mises en oeuvre et de réalisations. C'est ainsi qu'il est possible de mesurer les pathologies buccales qu'elles intéressent les tissus durs et les tissus mous.

Le dispositif selon l'invention permet de visualiser et de mesurer des parties visibles et invisibles durant l'acte clinique prises dans son champ d'application, et répond à de nombreuses demandes en matière de coût, de convivialité, d'aide à la mesure ou à l'imagerie diagnostique en dentisterie.

Ce système peut être par exemple appliqué, dans une forme évolutive, à toute acquisition 3D nécessitant une manipulation rapide et précise obligeant l'opérateur à ne pas quitter des yeux son champ de travail, d'analyse et/ou de mesure. C'est le cas des travaux effectué sur toutes les parties du corps humain, l'acquisition de données nécessitant de ne pas être dérangé par des mouvements soudains du patient, les actions rapides comme les gestes sportifs ou les procédures de production industrielles tout particulièrement en milieu hostile. Il est ainsi possible de suivre et informer l'opérateur en temps réel ou quasi réel tout en lui permettant de ne pas quitter la scène des yeux et en lui affichant des informations complémentaires.

Il ressort de la description qui précède que la présente invention, répond parfaitement aux problèmes posés, en ce sens, qu'elle apporte une réelle réponse à la visualisation des zones visibles et invisible et plus particulièrement à leurs réunions dans un même référentiel permettant la visualisation de l'objet complémentarisé directement en bouche sur le site clinique. Elle permet une analyse anatomique immédiate et pathologique des pathologies de la gencive et des tissus sous-jacents. Il ressort aussi de cette description, qu'elle permet, de résoudre les problèmes fondamentaux, comme le contrôle de l'acte clinique, ce d'autant qu'aucune méthode alternative n'a été proposée. Il va de soi que l'invention ne se limite pas au seul mode de mise en oeuvre de ce procédé, ni aux seules formes d'exécution de dispositif pour la mise en oeuvre de ce procédé, écrites ci-dessus à titre d'exemple. Elle embrasse, au contraire, toutes les variantes de mises en oeuvre et de réalisations. C'est ainsi, notamment, qu'il est possible de mesurer les pathologies buccales qu'elles intéressent les tissus durs et les tissus mous.

L'invention peut être utilisée pour réaliser des actes médicaux ou infirmiers : il est possible que le dispositif de visualisation aide pour le repérage des éléments anatomiques pour une piqûre sous cutanée, une piqure intraveineuse ou la pose d'un cathéter ; il est également possible qu'il aide pour l'étude des épaisseurs de peau et de gencive entre l'os et la surface de la peau ou de la gencive.

**Revendications**

1. Dispositif de visualisation de l'intérieur d'une bouche d'un patient (13), le dispositif de visualisation comprenant un émetteur de rayons pénétrants (2) adapté à prendre une vue d'une partie interne (14, 22, 23, 27, 28, 31, 32, 33, 34, 35, 36, 37, 50, 51, 62, 63) située sous une surface externe d'un organe disposé dans la bouche, une paire de lunettes à réalité augmentée (1) ayant d'une part, un verre optique au travers duquel un utilisateur (6) de la paire de lunettes (1) peut voir l'intérieur de la bouche, et, d'autre part, une caméra de visualisation (9) adaptée à prendre en image ce que l'utilisateur (6) voit au travers du verre optique, une unité centrale (3) étant adaptée à corréler des premières images correspondantes à celles prises par la caméra de visualisation (9) avec des secondes images correspondantes à celles prises par l'émetteur de rayons pénétrants (2), **caractérisé en ce que** l'unité centrale est adaptée en sorte que soit projetée sur ledit verre optique une corrélation d'images constituée :

   - de ladite vue d'une partie interne,
   - de la visualisation directe au travers desdites lunettes et
   - de ladite image prise par la caméra (9),

   ladite partie interne étant ainsi visible à travers ledit verre optique, en superposition à la visualisation directe et à ladite image prise par la caméra (9).

2. Dispositif de visualisation selon la revendication 1, **caractérisé en ce que** l'unité centrale (3) est adaptée à orienter les secondes images en fonction de l'orientation de la paire de lunettes à réalité augmentée (1).

3. Dispositif de visualisation selon l'une des revendications 1 et 2, **caractérisé en ce que** l'unité centrale (3) est adaptée à projeter sur le verre optique la corrélation des premières images avec les secondes images.

4. Dispositif de visualisation selon la revendication 3, **caractérisé en ce que** l'unité centrale (3) est adaptée à projeter sur le verre optique, sur commande de l'utilisateur (6), des images d'une sélection de composants anatomiques de l'organe pris par l'émetteur de rayons pénétrants (2).

5. Dispositif de visualisation selon l'une des revendications 1 à 4, **caractérisé en ce qu'**il comprend une caméra de prise d'empreinte optique (4) adaptée à prendre une empreinte optique d'une surface externe d'un organe disposé dans la bouche, l'unité centrale (3) étant adaptée à corréler des troisièmes images correspondantes à celles prises par la caméra de prise d'empreinte optique (4) avec les premières images.

6. Dispositif de visualisation selon l'une des revendications 1 à 5, **caractérisé en ce que** la corrélation des images réalisée par l'unité centrale (3) est une superposition des images sur le verre optique.

7. Dispositif de visualisation selon l'une des revendications 1 à 6, **caractérisé en ce que** l'unité centrale (3) est adaptée, sur commande de l'utilisateur (6), à modifier le contraste et la transparence des images qu'il traite.

8. Dispositif de visualisation selon l'une des revendications 1 à 7, **caractérisé en ce que** l'émetteur de rayons pénétrants (2) est adapté à transmettre numériquement à l'unité centrale (3) les images qu'il prend.

9. Dispositif de visualisation selon l'une des revendications 1 à 7, **caractérisé en ce qu'**il comprend un dispositif de numérisation adapté à numériser les images non numériques émises par l'émetteur de rayons pénétrants (2) et à transmettre les images numérisées à l'unité centrale (3).

10. Dispositif de visualisation selon l'une des revendications 1 à 9, **caractérisé en ce que** l'unité centrale (3) est adaptée à projeter sur le verre optique des informations supplémentaires relatives au patient (13).

11. Dispositif de visualisation selon la revendication 10, **caractérisé en ce que** les informations supplémentaires relatives au patient (13) comprennent des données à respecter pour la réalisation d'une prothèse dentaire.

12. Dispositif de visualisation selon l'une des revendications 10 ou 11, **caractérisé en ce qu'**il comprend au moins un instrument périphérique relié à l'unité centrale (3) et adapté à capter des informations supplémentaires relatives au patient (13) .

13. Dispositif de visualisation selon la revendication 12, **caractérisé en ce que** l'un des instruments périphérique permet soit de capter l'occlusion statique et les mouvements mandibulaires, soit de capter la couleur des dents, soit de capter la forme du visage, soit de capter les données physiologiques du patient (13).

14. Dispositif de visualisation selon l'une des revendications 1 à 13, **caractérisé en ce qu'**il comprend un microphone (11) adapté à capter des ordres de commande provenant de l'utilisateur (6) et à les transmettre à l'unité centrale (3).

15. Dispositif de visualisation selon l'une des revendications 1 à 14, **caractérisé en ce que** la paire de lunettes à réalité augmentée (1) comprend un instrument de repérage spatial.

16. Dispositif de visualisation selon l'une des revendications 1 à 15, **caractérisé en ce qu'**il comprend un système d'éclairage (5) adapté à éclairer l'organe disposé dans la bouche.

17. Dispositif de visualisation selon la revendication 16, **caractérisé en ce que** le système d'éclairage (5) comprend des diodes électroluminescentes dont la longueur d'onde est adaptée à permettre le repérage de pathologies.

18. Dispositif de visualisation selon l'une des revendications 1 à 17, **caractérisé en ce que** l'unité centrale (3) est adaptée à projeter sur un écran déporté (7) des images relatives à l'organe disposé dans la bouche.

19. Ensemble de suivi médical **caractérisé en ce qu'**il comprend un dispositif de visualisation selon l'une des revendications 1 à 18 et un instrument de traitement médical (46, 47, 48, 49, 54, 58, 59) qui comporte, d'une part, un outil (46, 49, 58, 59) adapté à traiter des composants anatomiques d'un organe avec lesquels il est en contact, et, d'autre part, un repère (47) adapté à être repéré spatialement par le dispositif de visualisation lors du traitement des composants anatomiques, l'unité centrale (3) étant adaptée à connaître les dimensions de l'outil (46, 49, 58, 59) et la distance séparant l'outil (46, 49, 58, 59) du repère (47), et à déterminer la position de l'outil (46, 49, 58, 59) dans l'organe lors du traitement.

20. Ensemble de suivi médical selon la revendication 19, **caractérisé en ce que** l'unité centrale (3) est adaptée à réaliser des quatrièmes images qui représentent l'outil (46, 49, 58, 59) utilisé pour le traitement, à les corréler avec les secondes images, et à projeter la corrélation de façon à permettre la visualisation de l'outil (46, 49, 58, 59) dans l'organe en cours de traitement.

**EP 3 253 279 B1**

21. Ensemble de suivi médical selon l'une des revendications 19 et 20, **caractérisé en ce que**, la longueur du déplacement de l'outil (46, 49, 58, 59) étant égale à la longueur du déplacement du repère (47), l'unité centrale (3) est adaptée à déterminer la direction et le sens du déplacement de l'outil (46, 49, 58, 59) par rapport aux composants anatomiques avec lesquels il est en contact, la direction et le sens du déplacement de l'outil (46, 49, 58, 59) étant soit égaux à la direction et au sens du déplacement du repère (47) si l'outil (46, 49, 58, 59) est indéformable par rapport à ces composants anatomiques, soit déterminés par le relief de ces composants anatomiques si l'outil (46, 49, 58, 59) est déformable par rapport à ces derniers.

22. Ensemble de suivi médical selon l'une des revendications 19 à 21, **caractérisé en ce que** l'unité centrale (3) est adaptée à déterminer le mouvement idéal (55) de l'outil (46, 49, 58, 59) utilisé pour la réalisation d'un traitement.

23. Ensemble de suivi médical selon la revendication 22, **caractérisé en ce que** l'unité centrale (3) est adaptée à guider l'utilisateur (6) pour que l'outil (46, 49, 58, 59) utilisé suive le mouvement idéal (55).

24. Ensemble de suivi médical selon la revendication 23, **caractérisé en ce que** le guidage de l'utilisateur (6) est réalisé par l'affichage du mouvement idéal (55) sur le verre optique corrélé avec les secondes images.

25. Ensemble de suivi médical selon l'une des revendications 23 et 24, **caractérisé en ce que** le guidage de l'utilisateur (6) est réalisé par l'émission d'un signal sonore dépendant de la position de l'outil (46, 49, 58, 59) utilisé.

26. Ensemble de suivi médical selon l'une des revendications 19 à 25, **caractérisé en ce que** l'unité centrale (3) est adaptée à recevoir un identifiant propre à de chaque outil (46, 49, 58, 59) pouvant être utilisé et à déterminer l'outil (46, 49, 58, 59) utilisé.

27. Ensemble de suivi médical selon la revendication 26, **caractérisé en ce que** l'unité centrale (3) comprend une bibliothèque associant à chaque outil (46, 49, 58, 59) pouvant être utilisé l'identifiant qui lui est propre.

28. Ensemble de réalisation de prothèses comprenant un dispositif de visualisation selon l'une des revendications 1 à 18 et une machine à commande numérique (12) commandée par l'unité centrale (3) pour la réalisation d'une prothèse relative à l'organe disposé dans la bouche.


**Patentansprüche**

1. Visualisierungsvorrichtung für das Innere eines Mundes eines Patienten (13), wobei die Visualisierungsvorrichtung einen Sender durchdringender Strahlen (2) umfasst, der dazu ausgelegt ist, eine Ansicht eines inneren Teils (14, 22, 23, 27, 28, 31, 32, 33, 34, 35, 36, 37, 50, 51, 62, 63) aufzunehmen, die sich unter einer Außenfläche eines im Mund angeordneten Organs befindet, eine Augmented-Reality-Brille (1), die einerseits ein optisches Glas aufweist, durch das ein Benutzer (6) der Brille (1) das Innere des Mundes sehen kann, und andererseits eine Visualisierungskamera (9), die dazu ausgelegt ist, ein Bild von dem aufzunehmen, was der Benutzer (6) durch das optische Glas sieht, wobei eine Zentraleinheit (3) dazu ausgelegt ist, erste Bilder, die den von der Visualisierungskamera (9) aufgenommenen Bildern entsprechen, mit zweiten Bildern, die den von dem Sender durchdringender Strahlen (2) aufgenommenen Bildern entsprechen, zu korrelieren, **dadurch gekennzeichnet, dass** die Zentraleinheit dazu ausgelegt ist, dass auf das optische Glas eine Korrelation von Bildern projiziert wird, bestehend aus:

   - der Ansicht eines inneren Teils,
   - der direkten Visualisierung durch die Brille und
   - dem von der Kamera (9) aufgenommenen Bild,

   wobei der innere Teil somit in Überlagerung mit der direkten Visualisierung und dem von der Kamera (9) aufgenommenen Bild durch das optische Glas sichtbar wird.

2. Visualisierungsvorrichtung nach Anspruch 1, **dadurch gekennzeichnet, dass** die Zentraleinheit (3) dazu ausgelegt ist, die zweiten Bilder in Abhängigkeit von der Ausrichtung der Augmented-Reality-Brille (1) auszurichten.

3. Visualisierungsvorrichtung nach einem der Ansprüche 1 oder 2, **dadurch gekennzeichnet, dass** die Zentraleinheit (3) dazu ausgelegt ist, die Korrelation der ersten Bilder mit den zweiten Bildern auf das optische Glas zu projizieren.

4. Visualisierungsvorrichtung nach Anspruch 3, **dadurch gekennzeichnet, dass** die Zentraleinheit (3) dazu ausgelegt ist, auf Befehl des Benutzers (6) Bilder einer Auswahl von anatomischen Bestandteilen des Organs, die von dem Sender durchdringender Strahlen (2) aufgenommen werden, auf das optische Glas zu projizieren.

5. Visualisierungsvorrichtung nach einem der Ansprüche 1 bis 4, **dadurch gekennzeichnet, dass** sie eine optische Abdruckaufnahmekamera (4) umfasst, die dazu ausgelegt ist, einen optischen Abdruck einer Außenfläche eines im Mund angeordneten Organs aufzunehmen, wobei die Zentraleinheit (3) dazu ausgelegt ist, dritte Bilder, die den von der optischen Abdruckkamera (4) aufgenommenen Bildern entsprechen, mit den ersten Bildern zu korrelieren.

6. Visualisierungsvorrichtung nach einem der Ansprüche 1 bis 5, **dadurch gekennzeichnet, dass** die von der Zentraleinheit (3) durchgeführte Korrelation der Bilder eine Überlagerung der Bilder auf dem optischen Glas ist.

7. Visualisierungsvorrichtung nach einem der Ansprüche 1 bis 6, **dadurch gekennzeichnet, dass** die Zentraleinheit (3) auf Befehl des Benutzers (6) dazu ausgelegt ist, den Kontrast und die Transparenz der von ihr verarbeiteten Bilder zu verändern.

8. Visualisierungsvorrichtung nach einem der Ansprüche 1 bis 7, **dadurch gekennzeichnet, dass** der Sender durchdringender Strahlen (2) dazu ausgelegt ist, die von ihm aufgenommenen Bilder digital an die Zentraleinheit (3) zu übertragen.

9. Visualisierungsvorrichtung nach einem der Ansprüche 1 bis 7, **dadurch gekennzeichnet, dass** sie eine Digitalisierungsvorrichtung umfasst, die dazu ausgelegt ist, die vom Sender durchdringender Strahlen (2) ausgesendeten, nicht-digitalen Bilder zu digitalisieren und die digitalisierten Bilder an die Zentraleinheit (3) zu übertragen.

10. Visualisierungsvorrichtung nach einem der Ansprüche 1 bis 9, **dadurch gekennzeichnet, dass** die Zentraleinheit (3) dazu ausgelegt ist, zusätzliche Informationen über den Patienten (13) auf das optische Glas zu projizieren.

11. Visualisierungsvorrichtung nach Anspruch 10, **dadurch gekennzeichnet, dass** die zusätzlichen Informationen über den Patienten (13) Daten umfassen, die bei der Herstellung eines Zahnersatzes zu beachten sind.

12. Visualisierungsvorrichtung nach einem der Ansprüche 10 oder 11, **dadurch gekennzeichnet, dass** sie mindestens ein peripheres Instrument umfasst, das mit der Zentraleinheit (3) verbunden und dazu ausgelegt ist, zusätzliche Informationen über den Patienten (13) zu erfassen.

13. Visualisierungsvorrichtung nach Anspruch 12, **dadurch gekennzeichnet, dass** eines der peripheren Instrumente so konfiguriert ist, dass es entweder die statische Okklusion und die Unterkieferwege erfasst, oder die Zahnfarbe erfasst, oder die Gesichtsform erfasst oder die physiologischen Daten des Patienten erfasst (13).

14. Visualisierungsvorrichtung nach einem der Ansprüche 1 bis 13, **dadurch gekennzeichnet, dass** sie ein Mikrophon (11) umfasst, das dazu ausgelegt ist, Steuerbefehle vom Benutzer (6) zu erfassen und diese an die Zentraleinheit (3) zu übertragen.

15. Visualisierungsvorrichtung nach einem der Ansprüche 1 bis 14, **dadurch gekennzeichnet, dass** die Augmented-Reality-Brille (1) ein Instrument zur räumlichen Ortung umfasst.

16. Visualisierungsvorrichtung nach einem der Ansprüche 1 bis 15, **dadurch gekennzeichnet, dass** sie ein Beleuchtungssystem (5) umfasst, das dazu ausgelegt ist, das im Mund angeordnete Organ zu beleuchten.

17. Visualisierungsvorrichtung nach Anspruch 16, **dadurch gekennzeichnet, dass** das Beleuchtungssystem (5) Leuchtdioden umfasst, deren Wellenlänge dazu ausgelegt ist, die Erkennung von Pathologien zu ermöglichen.

18. Visualisierungsvorrichtung nach einem der Ansprüche 1 bis 17, **dadurch gekennzeichnet, dass** die Zentraleinheit (3) dazu ausgelegt ist, Bilder, die sich auf das im Mund angeordnete Organ beziehen, auf einen entfernten Bildschirm (7) zu projizieren.

19. Medizinische Überwachungsanordnung, **dadurch gekennzeichnet, dass** sie eine Visualisierungsvorrichtung nach einem der Ansprüche 1 bis 18 und ein medizinisches Behandlungsinstrument (46, 47, 48, 49, 54, 58, 59) umfasst, das einerseits ein Werkzeug (46, 49, 58, 59) beinhaltet, das dazu ausgelegt ist, anatomische Bestandteile eines Organs zu behandeln, mit denen es in Kontakt steht, und andererseits, eine Markierung (47), die dazu ausgelegt ist, bei der Behandlung der anatomischen Bestandteile von der Visualisierungsvorrichtung räumlich lokalisiert zu werden, wobei die Zentraleinheit (3) dazu ausgelegt ist, die Abmessungen des Werkzeugs (46, 49, 58, 59) und den Abstand, der das Werkzeug (46, 49, 58, 59) von der Markierung (47) trennt, zu kennen und bei der Behandlung die Position des Werkzeugs (46, 49, 58, 59) im Organ zu bestimmen.

20. Medizinische Überwachungsanordnung nach Anspruch 19, **dadurch gekennzeichnet, dass** die Zentraleinheit (3) dazu ausgelegt ist, vierte Bilder zu erstellen, die das zur Behandlung verwendete Werkzeug (46, 49, 58, 59) darstellen, diese mit den zweiten Bildern zu korrelieren und die Korrelation so zu projizieren, dass eine Visualisierung des Werkzeugs (46, 49, 58, 59) in dem zu behandelnden Organ ermöglicht wird.

21. Medizinische Überwachungsanordnung nach einem der Ansprüche 19 oder 20, **dadurch gekennzeichnet, dass**, da die Länge der Bewegung des Werkzeugs (46, 49, 58, 59) gleich der Länge der Bewegung der Markierung (47) ist, die Zentraleinheit (3) dazu ausgelegt ist, die Bewegungsrichtung und -orientierung des Werkzeugs (46, 49, 58, 59) in Bezug auf die anatomischen Bestandteile, mit denen es in Kontakt steht, zu bestimmen, wobei die Bewegungsrichtung und -orientierung des Werkzeugs (46, 49, 58, 59) entweder gleich der Bewegungsrichtung und -orientierung der Markierung (47) ist, sofern das Werkzeug (46, 49, 58, 59) in Bezug auf diese anatomischen Bestandteile nicht verformbar ist, oder durch die Kontur dieser anatomischen Bestandteile bestimmt wird, sofern das Werkzeug (46, 49, 58, 59) in Bezug auf jene verformbar ist.

22. Medizinische Überwachungsanordnung nach einem der Ansprüche 19 bis 21, **dadurch gekennzeichnet, dass** die Zentraleinheit (3) dazu ausgelegt ist, den idealen Weg (55) des Werkzeugs (46, 49, 58, 59) zu bestimmen, das zur Durchführung einer Behandlung verwendet wird.

23. Medizinische Überwachungsanordnung nach Anspruch 22, **dadurch gekennzeichnet, dass** die Zentraleinheit (3) dazu ausgelegt ist, den Benutzer (6) so zu führen, dass das verwendete Werkzeug (46, 49, 58, 59) dem idealen Weg (55) folgt.

24. Medizinische Überwachungsanordnung nach Anspruch 23, **dadurch gekennzeichnet, dass** die Führung des Benutzers (6) durch die Anzeige des idealen Wegs (55) auf dem optischen Glas erfolgt, die mit den zweiten Bildern korreliert ist.

25. Medizinische Überwachungsanordnung nach einem der Ansprüche 23 oder 24, **dadurch gekennzeichnet, dass** die Führung des Benutzers (6) durch das Aussenden eines akustischen Signals erfolgt, das von der Position des verwendeten Werkzeugs (46, 49, 58, 59) abhängt.

26. Medizinische Überwachungsanordnung nach einem der Ansprüche 19 bis 25, **dadurch gekennzeichnet, dass** die Zentraleinheit (3) dazu ausgelegt ist, von jedem Werkzeug (46, 49, 58, 59), das verwendet werden kann, eine eigene Kennung zu empfangen und das verwendete Werkzeug (46, 49, 58, 59) zu bestimmen.

27. Medizinische Überwachungsanordnung nach Anspruch 26, **dadurch gekennzeichnet, dass** die Zentraleinheit (3) eine Bibliothek umfasst, die jedem Werkzeug (46, 49, 58, 59), das verwendet werden kann, die ihm eigene Kennung zuordnet.

28. Anordnung zur Herstellung von Prothesen, umfassend eine Visualisierungsvorrichtung nach einem der Ansprüche 1 bis 18 und eine von der Zentraleinheit (3) gesteuerte digital gesteuerte Maschine (12) zur Herstellung einer Prothese in Bezug auf das im Mund angeordnete Organ.

**Claims**

1. Device for viewing the inside of a mouth of a patient (13), the viewing device comprising a penetrating ray emitter (2) capable of taking an image of an internal portion (14, 22, 23, 27, 28, 31, 32, 33, 34, 35, 36, 37, 50, 51, 62, 63) located under an outer surface of an organ arranged in the mouth, a pair of augmented reality glasses (1) having, on the one hand, an optical lens through which a user (6) of the pair of glasses (1) can view the inside of the mouth, and, on the other hand, a viewing camera (9) suitable for taking an image of what the user (6) sees through the optical lens, a central processing unit (3) capable of correlating first images corresponding to the images taken by the viewing camera (9) with second images corresponding to those taken by the penetrating ray emitter (2), **char-**

**acterized in that** the central processing unit is adapted so that a correlation of images is projected onto said optical lens, said correlation of images consisting of:

- said view of an internal portion,
- the direct view through said glasses and
- said image taken by the camera (9),

said internal portion thus being visible through said optical lens, superimposed on the direct view and on said image taken by the camera (9).

2. Viewing device according to claim 1, **characterized in that** the central processing unit (3) is suitable for orienting the second images according to the orientation of the pair of augmented reality glasses (1).

3. Viewing device according to either claim 1 or claim 2, **characterized in that** the central processing unit (3) is suitable for projecting, onto the optical lens, the correlation of the first images with the second images.

4. Viewing device according to claim 3, **characterized in that** the central processing unit (3) is suitable for projecting, onto the optical lens, at the request of the user (6), images of a selection of anatomical components of the organ taken by the penetrating ray emitter (2).

5. Viewing device according to any of claims 1 to 4, **characterized in that** it comprises a camera for taking optical impressions (4) which is suitable for taking an optical impression of an outer surface of an organ arranged in the mouth, the central processing unit (3) being suitable for correlating third images corresponding to the images taken by the optical impressions camera (4) with the first images.

6. Viewing device according to any of claims 1 to 5, **characterized in that** the correlation of the images that is produced by the central processing unit (3) is a superimposition of the images onto the optical lens.

7. Viewing device according to any of claims 1 to 6, **characterized in that** the central processing unit (3) is suitable for modifying, at the request of the user (6), the contrast and transparency of the images that said unit processes.

8. Viewing device according to any of claims 1 to 7, **characterized in that** the penetrating ray emitter (2) is suitable for digitally transmitting, to the central processing unit (3), the images that said emitter takes.

9. Viewing device according to any of claims 1 to 7, **characterized in that** it comprises a digitizing device suitable for digitizing the non-digital images produced by the penetrating ray emitter (2) and for transmitting the digitized images to the central processing unit (3).

10. Viewing device according to any of claims 1 to 9, **characterized in that** the central processing unit (3) is suitable for projecting, onto the optical lens, additional information pertaining to the patient (13).

11. Viewing device according to claim 10, **characterized in that** the additional information pertaining to the patient (13) comprises data which must be observed when producing a dental prosthesis.

12. Viewing device according to either claim 10 or claim 11, **characterized in that** it comprises at least one peripheral instrument connected to the central processing unit (3) and suitable for capturing additional information pertaining to the patient (13).

13. Viewing device according to claim 12, **characterized in that** one of the peripheral instruments makes it possible to capture the static occlusion and the mandibular movements of the patient, or to capture the color of the teeth, or to capture the shape of the face, or to capture the physiological data of the patient (13).

14. Viewing device according to any of claims 1 to 13, **characterized in that** it comprises a microphone (11) suitable for capturing control commands from the user (6) and transmitting said commands to the central processing unit (3).

15. Viewing device according to any of claims 1 to 14, **characterized in that** the pair of augmented reality glasses (1) comprises a spatial tracking instrument.

**16.** Viewing device according to any of claims 1 to 15, **characterized in that** it comprises a lighting system (5) suitable for illuminating the organ arranged in the mouth.

**17.** Viewing device according to claim 16, **characterized in that** the lighting system (5) comprises light-emitting diodes, the wavelength of which is suitable for identifying pathologies.

**18.** Viewing device according to any of claims 1 to 17, **characterized in that** the central processing unit (3) is suitable for projecting, onto a remote display (7), images relating to the organ arranged in the mouth.

**19.** Medical monitoring assembly **characterized in that** it comprises a viewing device according to any of claims 1 to 18 and a medical treatment instrument (46, 47, 48, 49, 54, 58, 59) which comprises, on the one hand, a tool (46, 49, 58, 59) suitable for treating the anatomical components of an organ with which said tool is in contact, and, on the other hand, a marker (47) suitable for being spatially tracked by the viewing device during the treatment of the anatomical components, the central processing unit (3) being suitable for recognizing the dimensions of the tool (46, 49, 58, 59) and the distance separating the tool (46, 49, 58, 59) from the marker (47), and for determining the position of the tool (46, 49, 58, 59) in the organ during the treatment.

**20.** Medical monitoring assembly according to claim 19, **characterized in that** the central processing unit (3) is suitable for producing fourth images which depict the tool (46, 49, 58, 59) used for the treatment, and for correlating the fourth images with the second images, and for projecting the correlation so as to allow the tool (46, 49, 58, 59) to be viewed in the organ during the treatment.

**21.** Medical monitoring assembly according to either claim 19 or claim 20, **characterized in that**, since the length of movement of the tool (46, 49, 58, 59) is equal to the length of movement of the marker (47), the central processing unit (3) is suitable for determining the direction and the direction of movement of the tool (46, 49, 58, 59) relative to the anatomical components with which said tool is in contact, the direction and the direction of movement of the tool (46, 49, 58, 59) being either equal to the direction and the direction of movement of the marker (47) if the tool (46, 49, 58, 59) is non-deformable relative to said anatomical components, or determined by the relief of said anatomical components if the tool (46, 49, 58, 59) is deformable relative to said anatomical components.

**22.** Medical monitoring assembly according to any of claims 19 to 21, **characterized in that** the central processing unit (3) is suitable for determining the optimal movement (55) of the tool (46, 49, 58, 59) used for performing treatment.

**23.** Medical monitoring assembly according to claim 22, **characterized in that** the central processing unit (3) is suitable for guiding the user (6) so that the tool (46, 49, 58, 59) used follows the optimal movement path (55).

**24.** Medical monitoring assembly according to claim 23, **characterized in that** the user (6) is guided by displaying, on the optical lens, the ideal movement path (55) correlated with the second images.

**25.** Medical monitoring assembly according to either claim 23 or claim 24, **characterized in that** the user (6) is guided by emitting an audible signal dependent on the position of the tool (46, 49, 58, 59) used.

**26.** Medical monitoring assembly according to any of claims 19 to 25, **characterized in that** the central processing unit (3) is suitable for receiving an identifier specific to each tool (46, 49, 58, 59) that can be used and for determining the tool (46, 49, 58, 59) used.

**27.** Medical monitoring assembly according to claim 26, **characterized in that** the central processing unit (3) comprises a library associating, with each tool (46, 49, 58, 59) that can be used, the identifier which is specific thereto.

**28.** Assembly for producing prostheses, comprising a viewing device according to any of claims 1 to 18 and a digital machine (12) controlled by the central processing unit (3), for producing a prosthesis relating to the organ arranged in the mouth.

FIG. 1

FIG. 2

Périphériques pour la numérisation du
volume invisible (Rx, IRM, OCT, US ...)

2

Lunettes d'affichage,de
visualisation et numérisation
de la partie visible

1

Unité
Centrale
et softwares

3

Caméra endobuccale 3D ou
porte empreinte optique
précise

4

Eclairage projeté structuré
ou non struturé

5

FIG. 3

FIG. 4a

FIG. 4b

FIG. 4c

FIG. 4d

18

14

16

15

17

FIG. 4e

FIG. 4f

FIG. 4g

FIG. 7

FIG. 5d

FIG. 5c

FIG. 5a

FIG. 5b

22

FIG. 6a

FIG. 6b

FIG. 6c

FIG. 6d

FIG. 8a

FIG. 8b

60
64
27
61

62

63

FIG. 8c

Prise de vue à l'aide d'un périphérique permettant le captage et la numérisation des parties invisibles (2) avec des rayonnements ionisants (Rx ...) ou non (IRM ...)

66

Unité Centrale et softwares (3)

65

67

1. Création d'un nuage de points et d'une modélisation de la partie invisible (sous gingivales) renfermant une zone spécifique

Visialisation de la bouche, prise d'empreinte optique peu précise avec les caméras (9) sur les lunettes (1)

70

68

2. Création d'un nuage de points et d'une modélisation de la partie visible (correspondant à la vision directe du clinicien)

69

Eventuellement renforcement de la précision de prise d'empreinte de la partie visible avec scanner buccale (4)

72

73

3. renforcement du nuage de point donc de la précision et de la modélisation grâce au scanner endobuccale (4)

71

Sélection d'un éclairage spécifique pour les zones pathologiques (5)

75

4. sélection de recherche d'une pathologie scanner endobuccale (4)

74

76

Visialisation enrichie au travers des lunettes (1) permettant de voir le model de complémentarité renfermant la partie visible et invisible en sur impression sur la vision directe.

78

5. Mise en correspondance des nuages de points globaux et précis et création du model de complémentarité (20)

77

IHM
Détermination des paramètres d'affichage et sélection des indications complémentaires

80

79

FIG. 9

37

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5092022 A
- US 4952149 A
- US 0109559 A
- US 7372642 B
- US 8520925 B
- US 6006126 A

- FR 8307840
- FR 9208128
- EP 1756771 A
- EP 0600128 A
- FR 1454774

**Littérature non-brevet citée dans la description**

- *Appl. Opt.,* 1988, vol. 27

- *SPIE,* 1981, vol. 283